# EUROPEAN PATENT SPECIFICATION

(11) **EP 3 630 001 B1**
(45) Date of publication and mention of the grant of the patent: **15.04.2026**
(21) Application number: 18730448.0
(22) Date of filing: 16.05.2018
(51) Int. Cl.: A61C 17/02, A61C 17/028

(54) **ORAL CARE CLEANING SYSTEM UTILIZING ENTRAINED FLUID**
MUNDPFLEGEREINIGUNGSSYSTEM MIT VERWENDUNG VON MITGEFÜHRTER FLÜSSIGKEIT
SYSTÈME DE NETTOYAGE DE SOIN BUCCAL UTILISANT UN FLUIDE ENTRAÎNÉ

(30) Priority: 01.06.2017 US 201715611031
(43) Date of publication of application: 08.04.2020
(73) Proprietor: Kenvue Brands LLC, Summit, NJ 07901 (US)
(72) Inventor: DORWARD, Brian, Great Meadows, New Jersey 07838 (US); FOURRE, Tara, Skillman, New Jersey 08558 (US); MCDONOUGH, Justin, Skillman, New Jersey 08558 (US); MIKSA, Davide, Skillman, New Jersey 08558 (US); SEO, Jin, Skillman, New Jersey 08558 (US); SHARMA, Deepak, Skillman, New Jersey 08558 (US)
(74) Representative: Carpmaels & Ransford LLP
(86) International application number: PCT/IB2018/053447
(87) International publication number: WO 2018/220463

(56) References cited:
- US-A- 3 379 192
- US-A1- 2011 027 746
- US-A1- 2012 077 143
- US-A1- 2012 107 765
- US-A1- 2017 056 143

## Description

### FIELD OF THE INVENTION

The present invention relates to oral care systems suitable for in-home use to provide a beneficial effect to the oral cavity of a mammal.

### BACKGROUND OF THE INVENTION

In addition to regular professional dental checkups, daily oral hygiene is generally recognized as an effective preventative measure against the onset, development, and/or exacerbation of periodontal disease, gingivitis and/or tooth decay. Unfortunately, however, even the most meticulous individuals dedicated to thorough brushing and flossing practices often fail to reach, loosen and remove deep-gum and/or deep inter-dental food particulate, plaque or biofilm. Most individuals have professional dental cleanings biannually to remove tarter deposits.

For many years products have been devised to facilitate the simple home cleaning of teeth, although as yet a single device which is simple to use and cleans all surfaces of a tooth and/or the gingival or sub-gingival areas simultaneously is not available. The conventional toothbrush is widely utilized, although it requires a significant input of energy to be effective and, furthermore, a conventional toothbrush cannot adequately clean the inter-proximal areas of the teeth. Cleaning of the areas between teeth currently requires the use of floss, pick, or some such other additional device apart from a toothbrush.

Electric toothbrushes have achieved significant popularity and, although these reduce the energy input required to utilize a toothbrush, they are still inadequate to ensure proper inter-proximal tooth cleaning. Oral irrigators are known to clean the inter-proximal area between teeth. However, such devices have a single jet which must be directed at the precise inter-proximal area involved in order to remove debris. These water pump type cleaners are therefore typically only of significant value in connection with teeth having braces thereupon which often trap large particles of food. It will be appreciated that if both debris and plaque are to be removed from teeth, at present a combination of a number of devices must be used, which is extremely time consuming and inconvenient.

In addition, in order for such practices and devices to be effective, a high level of consumer compliance with techniques and/or instructions is required. The user-to-user variation in time, cleaning/treating formula, technique, etc., will affect the cleaning of the teeth.

The present invention ameliorates one or more of the above mentioned disadvantages with existing oral hygiene apparatus and methods, or at least provides the market with an alternative technology that is advantageous over known technology, and also may be used to ameliorate a detrimental condition or to improve cosmetic appearance of the oral cavity.

US 2017/056143 A1 describes an apparatus for cleaning the oral cavity, which comprises: a body including insertion slots into which the teeth of a user can be inserted, a plurality of injection holes and a plurality of aspiration holes formed on the inner wall of the insertion slots; a feeding tube for supplying cleaning liquid to the plurality of injection holes; a discharge tube through which the cleaning liquid is externally discharged via the plurality of aspiration holes; and a switching member for switching the direction of injection and the direction of aspiration of the cleaning liquid, wherein the body includes a first inner space which communicates with the plurality of injection holes, and a second inner space which communicates with the plurality of aspiration holes.

US 2011/027746 A1 describes a device for directing a liquid onto a plurality of surfaces of the oral cavity, the device including a chamber for maintaining the liquid proximate the surfaces, where the chamber is defined by front, rear and base inner walls of the device and the front and rear inner walls each include a plurality of openings, the devices further including a first manifold and a second manifold, a first port and a second port; and means for providing an effective seal of the device within the oral cavity.

US 3 379 192 A describes a dental treatment device.

US 2012/077143 A1 describes a device for directing a liquid onto a plurality of surfaces of the oral cavity, the device including a handle, a neck, and a head, where the head includes a cleaning component including a chamber for maintaining the liquid proximate the surfaces, where the chamber is defined by front and rear sealing membranes, inner side walls and a base inner wall, and where the inner side walls each include a plurality of openings, the device further including a first manifold and a second manifold, a first port and a second port for conveying liquid; and means for providing an effective seal of the device within the oral cavity.
US 2012/107765 A1 describes an oral cleaning appliance that includes an assembly for generating low-pressure bursts of gas in the range of 2-7 bar, directed to a mixing chamber portion of the appliance.

### SUMMARY OF THE INVENTION

In one aspect, the present invention provides an oral care system comprising: an appliance comprising a first and second plurality of nozzles, said appliance configured to be held in the mouth of a user with said first and second plurality of nozzles in fluid communication with one or more surfaces of the user's oral cavity; a source of gas; a source of liquid, wherein said source of liquid and source of gas are separate sources; and a fluid controller for directing fluid to and removing fluid from said appliance; wherein said system is configured to produce a series of entrained fluid pulses from said sources of liquid and gas; direct such pulses through the first plurality of said nozzles in said appliance to said one or more surfaces of the user's oral cavity; and remove fluid from said appliance through the second plurality of nozzles to said fluid controller; wherein such system is configured such that gas from said source of gas is pulsed from said fluid controller to said first plurality of nozzles and liquid from said source of liquid flows or is pulsed into the gas pulsed from the fluid controller to form gas-entrained fluid between said fluid controller and said nozzles.

### BRIEF DESCRIPTION OF THE DRAWINGS

FIG. 1 is a schematic drawing of a first embodiment of a system according to the present invention;
FIG. 2 is a schematic drawing of a second embodiment of a system according to the present invention;
FIG. 3 is a schematic drawing of a third embodiment of a system according to the present invention;
FIG. 4 is a schematic drawing of a fourth embodiment of a system according to the present invention;
FIG. 5a is a schematic drawing of an embodiment of a reciprocating flow controller for use in a first embodiment of a system according to the present invention where the flow controller is in a first position;
FIG. 5b is a schematic drawing of the reciprocating flow controller of FIG. 5a in a second position;
FIG. 6a is a schematic drawing of an embodiment of a reciprocating flow controller for use in a second embodiment of a system according to the present invention where the flow controller is in a first position;
FIG. 6b is a schematic drawing of the reciprocating flow controller of FIG. 6a in a second position;
FIG. 7a is a schematic drawing of an embodiment of a reciprocating flow controller for use in a third embodiment of a system according to the present invention where the flow controller is in a first position;
FIG. 7b is a schematic drawing of the reciprocating flow controller of FIG. 7a in a second position;
FIG. 8a is a schematic drawing of an embodiment of a reciprocating flow controller for use in a fourth embodiment of a system according to the present invention where the flow controller is in a first position;
FIG. 8b is a schematic drawing of the reciprocating flow controller of FIG. 8a in a second position;
FIG. 9 is a top front perspective view of a first embodiment of an application tray for use with the present invention;
FIG. 10 is a top back view of the embodiment of the application tray of FIG. 9;
FIG. 11 is a bottom back view of the embodiment of the application tray of FIG. 9;
FIG. 12 is a cut-away view of the application tray of FIG. 9;
FIG. 13 is a top front perspective view of a second embodiment of an application tray for use with the present invention;
FIG. 14 is a bottom rear perspective view of the embodiment of the application tray of FIG. 13;
FIG. 15 is a cross-sectional view of a mammalian tooth in the gums of the oral cavity.
FIG. 16 is a cross-sectional view of a section of a first embodiment of an appliance used according to the present invention;
FIG. 17 is a cross-sectional view of a section of a second embodiment of an appliance used according to the present invention; and
FIG. 18 is a cross-sectional view of a section of a third embodiment of an appliance used according to the present invention.

### DETAILED DESCRIPTION OF THE INVENTION

Applicants have recognized that entrained fluid may be used in accord with the systems and methods of the present invention to achieve significant beneficial effect in the oral cavity. According to certain embodiments of the present invention, applicants have discovered that entrained fluid may be used in a system comprising: (a) an appliance with a first and second plurality of nozzles, the appliance configured to be held in the mouth with the nozzles in fluid communication with one or more surfaces of the oral cavity, and (b) a fluid controller, to achieve beneficial effect by directing the entrained fluid through the first plurality of nozzles and into the oral cavity and removing fluid from the oral cavity through the second plurality of nozzles. In certain embodiments, such system may be further used to reciprocate entrained fluid back and forth across the surfaces of the oral cavity. For example, the fluid controller may operate to alternately: (i) direct entrained fluid through the first plurality of nozzles of the appliance while simultaneously applying vacuum to the second plurality of nozzles to remove fluid from the appliance; and (ii) direct entrained fluid through the second plurality of nozzles of the appliance while simultaneously applying vacuum to the first plurality of nozzles to remove fluid from the appliance.

According certain other embodiments, applicants have demonstrated the unexpected superior benefits associated with pulsing entrained fluid onto surfaces of the oral cavity through at least one nozzle using certain system parameters, such as, for example, liquid/gas pulse frequency ratio. These embodiments and others, as well as, the benefits associated therewith are further described herein below.

### Entrained Fluid

The term "entrained fluid" as used herein, refers broadly to gas-entrained liquid and liquid-entrained gas. As will be recognized by one of skill in the art, "gas-entrained liquid" refers to gas and liquid mixed such that small particles of the gas are dispersed in the liquid, while "liquid-entrained gas" refers to gas and liquid mixed such that small particles of the liquid are dispersed in the gas. In certain embodiments, the systems and methods of the present invention use liquid-entrained gas. In certain other embodiments, the systems and methods of the present invention use gas-entrained liquid.

According to certain embodiments, the present invention comprises a system configured to produce entrained fluid from a source of gas and a source of liquid, wherein the source of gas and source of liquid may be separate sources or may be combined to form a single fluid source of gas and liquid. The sources of gas and/or liquid in the present invention may each comprise a reservoir, tank, or other container suitable for holding the gas and/or liquid, or may comprise a closeable gas or liquid pipe, line, hose or other closeable continuous source of gas and/or liquid. In certain embodiments, the source of gas and source of liquid are combined to form a single fluid source of gas and liquid, including for example, a pressurized tank of the gas and liquid. In certain other embodiments, the source of gas and source of liquid are separate individual sources of gas and liquid selected from the group consisting of reservoirs, tanks, and the like.

As illustrated and described in further detail below, according to certain embodiments of the present invention, the gas or liquid may be entrained within the other to form entrained fluid that is subsequently introduced to and pulsed from a fluid controller to an appliance comprising nozzles in fluid communication with the oral cavity. For example, entrained fluid may be dispensed from a combined source of gas and liquid to a fluid controller which subsequently dispenses the entrained fluid to the appliance.

Alternately, gas and liquid provided from separate sources within the system may be combined in accord with the present invention to form entrained fluid that is then introduced to an appliance.

According to other embodiments, as further described below, the gas and liquid may be combined to form entrained fluid within the appliance. For example, gas from a gas source may be pulsed to an appliance while liquid from a liquid source is streamed or pulsed to the appliance. The entrained fluid is formed when the gas and liquid are combined in the appliance.

Any of a variety of suitable gases and liquids may be used to produce entrained fluids for use in accord with the present invention. Examples of suitable gases include, but are not limited to, air, nitrogen, argon, carbon dioxide, oxygen, nitrous oxide, nitric oxide, mixtures of two or more thereof and the like. In certain embodiments, the gas comprises air. In certain embodiments, the gas comprises nitrogen.

Any liquid suitable for producing entrained fluids for use in the oral cavity may be used in the present invention. In certain embodiments, the liquid will be, or will comprise, water. In certain embodiments, the composition comprises from about 60% to about 99.99% water, including from about 70% to about 95% water, from about 80% to 95% water, from about 60% to about 90% water, from about 60% to about 80% water, or from about 60% to about 75% water. In certain embodiments, the liquid may be a composition comprising alcohol. Any of a variety of alcohols represented by the formula R₄-OH, wherein R₄ is an alkyl group having from 2 to 6 carbons, may be used in the liquid of the present invention. Examples of suitable alcohols of formula R₄-OH include ethanol; n-propanol, iso-propanol; butanols; pentanols; hexanols, and combinations of two or more thereof, and the like. In certain embodiments, the alcohol is, or comprises, ethanol. In some embodiments, the alcohol may be present in the liquid composition in an amount of at least about 10.0% v/v of the total composition, or from about 10% to about 35% v/v of the total composition, or from about 15% to about 30% v/v of the total composition and may be from about 20% to about 25% v/v of the total composition. In other embodiments, the liquid composition may comprise a reduced level of alcohol. The phrase "reduced level" of alcohol means an amount of a R₄-OH alcohol of about 10% v/v or less, optionally of about 5% v/v or less, optionally of about 1% v/v or less, optionally of about 0.1% v/v or less by volume of the total composition. In certain embodiments, the compositions of the present invention are free of R₄-OH alcohols.

In certain embodiments, the liquid is a composition comprising at least one ingredient, or agent, effective for providing the beneficial effect sought, in an amount effective to provide the beneficial effect when contacted with the surfaces of the oral cavity. For example, the liquid may include, without limitation, an ingredient selected from the group consisting of antimicrobial agents, whitening agents, cleaning agents, mineralization agents, desensitizing agents, and combinations of two or more thereof.

Examples of suitable antimicrobial agents which may be employed include, without limitation, essential oils, including but not limited to menthol, thymol, eucalyptol, methyl salicylate, and combinations of two or more thereof, cetyl pyidium chloride (CPC), chlorhexidine, hexetidine, chitosan, triclosan, domiphen bromide, stannous fluoride, soluble pyrophosphates, metal oxides including but not limited to zinc oxide, peppermint oil, sage oil, sanguinaria, dicalcium dihydrate, aloe vera, polyols, protease, lipase, amylase, metal salts including but not limited to zinc citrate, and combinations of two or more thereof and the like.

Examples of suitable whitening agents include, but are not limited to, hydrogen peroxide, carbamide peroxide, other agents capable of generating hydrogen peroxide when applied to the teeth, abrasives such as silica, sodium bicarbonate, alumina, apatites, bioglass, and combinations of two or more thereof. In certain embodiments, the liquid composition comprises silica.

Any of a variety of additional surfactants may be used in a liquid composition present invention. Suitable surfactants may include anionic, non-ionic, cationic, amphoteric, zwitterionic surfactants, and combinations of two or more thereof. Examples of suitable surfactants are disclosed, for example, in U.S. Pat. No. 7,417,020 to Fevola, et al.

In certain embodiments, the compositions of the present invention comprise a non-ionic surfactant. Those of skill in the art will recognize that any of a variety of one or more non-ionic surfactants include, but are not limited to, compounds produced by the condensation of alkylene oxide groups (hydrophilic in nature) with an organic hydrophobic compound which may be aliphatic or alkyl-aromatic in nature. Examples of suitable nonionic surfactants include, but are not limited to, alkyl polyglucosides; alkyl glucose amines, block copolymers such as ethylene oxide and propylene oxide copolymers e.g. Poloxamers; ethoxylated hydrogenated castor oils available commercially for example under the trade name CRODURET (Croda Inc., Edison, NJ); alkyl polyethylene oxide e.g. Polysorbates, and/or; fatty alcohol ethoxylates; polyethylene oxide condensates of alkyl phenols; products derived from the condensation of ethylene oxide with the reaction product of propylene oxide and ethylene diamine; ethylene oxide condensates of aliphatic alcohols; long chain tertiary amine oxides; long chain tertiary phosphine oxides; long chain dialkyl sulfoxides; and mixtures thereof.

Exemplary non-ionic surfactants are selected from the group known as poly(oxyethylene)-poly(oxypropylene) block copolymers. Such copolymers are known commercially as poloxamers and are produced in a wide range of structures and molecular weights with varying contents of ethylene oxide. These non-ionic poloxamers are non-toxic and acceptable as direct food additives. They are stable and readily dispersible in aqueous systems and are compatible with a wide variety of formulations and other ingredients for oral preparations. These surfactants should have an HLB (Hydrophilic-Lipophilic Balance) of between about 10 and about 30 and preferably between about 10 and about 25. By way of example, non-ionic surfactants useful in this invention include the poloxamers identified as poloxamers 105, 108, 124, 184, 185, 188, 215, 217, 234, 235, 237, 238, 284, 288, 333, 334, 335, 338, 407, and combinations of two or more thereof. In certain preferred embodiments, the composition comprises poloxamer 407.

In certain embodiments, the compositions of the claimed invention comprise less than about 9% of non-ionic surfactant, less than 5%, or less than 1.5%, or less than 1%, or less than 0.8, less than 0.5%, less than 0.4%, or less than 0.3% of non-ionic surfactants. In certain embodiments, the composition of the present invention is free of non-ionic surfactants.

In certain embodiments, the compositions of the present invention also contain at least one alkyl sulfate surfactant. In certain embodiments, suitable alkyl sulfate surfactants include, but are not limited to sulfated C₈ to C₁₈, optionally sulfated C₁₀ to C₁₆ even numbered carbon chain length alcohols neutralized with a suitable basic salt such as sodium carbonate or sodium hydroxide and mixtures thereof such that the alkyl sulfate surfactant has an even numbered C₈ to C₁₈, optionally C₁₀ to C₁₆, chain length. In certain embodiments, the alkyl sulfate is selected from the group consisting of sodium lauryl sulfate, hexadecyl sulfate and mixtures thereof. In certain embodiments, commercially available mixtures of alkyl sulfates are used. In certain embodiments, the alkyl sulfate surfactant is present in the composition from about 0.001% to about 6.0% w/v, or optionally from about 0.1% to about 0.5% w/v of the composition.

Another suitable surfactant is one selected from the group consisting of sarcosinate surfactants, isethionate surfactants and taurate surfactants. Preferred for use herein are alkali metal or ammonium salts of these surfactants, such as the sodium and potassium salts of the following: lauroyl sarcosinate, myristoyl sarcosinate, palmitoyl sarcosinate, stearoyl sarcosinate and oleoyl sarcosinate. The sarcosinate surfactant may be present in the compositions of the present invention from about 0.1% to about 2.5%, or from about 0.5% to about 2% by weight of the total composition.

Zwitterionic synthetic surfactants useful in the present invention include derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate or phosphonate.

The amphoteric surfactants useful in the present invention include, but are not limited to, derivatives of aliphatic secondary and tertiary amines in which the aliphatic radical can be a straight chain or branched and wherein one of the aliphatic substituents contains from about 8 to about 18 carbon atoms and one contains an anionic water-solubilizing group, e.g., carboxylate, sulfonate, sulfate, phosphate, or phosphonate. Examples of suitable amphoteric surfactants include, but are not limited alkylimino-diproprionates, alky lamphoglycinates (mono or di), alkylamphoproprionates (mono or di), alkylamphoacetates (mono or di), N-alkyl [3-aminoproprionic acids, alkylpolyamino carboxylates, phosphorylated imidazolines, alkyl betaines, alkylamido betaines, alkylamidopropyl betaines, alkyl sultaines, alkylamido sultaines, and mixtures thereof. In certain embodiments, the amphoteric surfactant is selected from the group consisting of alkylamidopropyl betaines, amphoacetates such as sodium auroamphoacetate and mixtures thereof. Mixtures of any of the above mentioned surfactants can also be employed. A more detailed discussion of anionic, nonionic and amphoteric surfactants can be found in U.S. Pat. No. 7,087,650 to Lennon; U.S. Pat. No. 7,084,104 to Martin et al.; U.S. Pat. No. 5,190,747 to Sekiguchi et al.; and U.S. Pat. No. 4,051,234, Gieske, et al..

In certain embodiments, the compositions of the claimed invention comprise less than about 9% of amphoteric surfactant, less than 5%, or less than 1.5%, or less than 1%, or less than 0.8, less than 0.5%, less than 0.4%, or less than 0.3% of amphoteric surfactants. In certain embodiments, the composition of the present invention is free of amphoteric surfactants.

Additional surfactants may be added with the alkyl sulfate surfactant to aid in solubilization of the essential oils provided such surfactants do not affect the bioavailability of the essential oils. Suitable examples include additional anionic surfactants, nonionic surfactants, amphoteric surfactants and mixtures thereof. However, in certain embodiments, the total surfactant concentration (including the alkyl sulfate surfactant alone or in combination with other surfactants) for mouth rinses of the present invention should not exceed or should about 9% or less, optionally, the total surfactant concentration should be about 5% or less, optionally about 1% or less, optionally about 0.5% or less w/w% of active surfactant by weight of the composition.

In certain embodiments, a sugar alcohol (humectant) is also added to the oral compositions of the present invention. The sugar alcohol solvent(s) may be selected from those multi-hydroxy-functional compounds that are conventionally used in oral and ingestible products. In certain embodiments, the sugar alcohol (s) should be nonmetabolized and non-fermentable sugar alcohol (s). In specific embodiments, the sugar alcohols include, but are not limited to sorbitol, glycerol, xylitol, mannitol, maltitol, inositol, allitol, altritol, dulcitol, galactitol, glucitol, hexitol, iditol, pentitol, ribitol, erythritol and mixtures thereof. Optionally, the sugar alcohol is selected from the group consisting of sorbitol and xylitol or mixtures thereof. In some embodiments, the sugar alcohol is sorbitol. In certain embodiments, the total amount of sugar alcohol (s), which are added to effectively aid in the dispersion or dissolution of the mouth rinse or other ingredients, should not exceed about 50% w/ of the total composition. Or, total amount of sugar alcohol should not exceed about 30% w/v of the total composition. Or, total amount of sugar alcohol should not exceed 25% w/v of the total composition. The sugar alcohol can be in an amount of from about 1.0% to about 24% w/v, or from about 1.5% to about 22% w/v, or from about 2.5% to about 20% w/v of the total composition.

In certain embodiments, a polyol solvent is added to the composition. The polyol solvent comprises a polyol or polyhydric alcohol selected from the group consisting of polyhydric alkanes (such as propylene glycol, glycerin, butylene glycol, hexylene glycol, 1,3-propanediol); polyhydric alkane esters (dipropylene glycol, ethoxydiglycol); polyalkene glycols (such as polyethylene glycol, polypropylene glycol) and mixtures thereof. In certain embodiments, the polyol solvent can be present in an amount of from 0% to about 40% w/v, or from about 0.5% to about 20% w/v, or from about 1.0% to about 10% w/v of the composition.

Sweeteners such as aspartame, sodium saccharin (saccharin), sucralose, stevia, acesulfame K and the like may be added for better taste in amounts of from about 0.0001% w/v to about 1.0% w/v. In certain preferred embodiments, the sweetener comprises sucralose.

In certain embodiments, the composition further comprises flavors or flavorants to modify or magnify the taste of the composition, or reduce or mask the sharp "bite" or "burn" of ingredients such as thymol. Suitable flavors include, but are not limited to, flavor oils such as oil of anise, anethole, benzyl alcohol, spearmint oil, citrus oils, vanillin and the like may be incorporated. Other flavors such as citrus oils, vanillin and the like may be incorporated to provide further taste variations. In these embodiments, the amount of flavor oil added to the composition can be from about 0.001% to about 5% w/v, or from about 0.01% to about 0.3% w/v of the total composition. The particular flavors or flavorants, and other taste improving ingredients, employed will vary depending upon the particular taste and feel desired. Those skilled in the art can select and customize these types of ingredients to provide the desired results.

In certain embodiments, acceptably approved food dyes may be used to provide a pleasing color to the compositions of the invention. These may be selected from, but not limited to, the long list of acceptable food dyes. Suitable dyes for this purpose include FD&C yellow #5, FD&C yellow #10, FD&C blue #1 and FD&C green #3. These are added in conventional amounts, typically in individual amounts of from about 0.00001% w/v to about 0.0008% w/v, or from about 0.000035% w/v to about 0.0005% w/v of the composition.

Other conventional ingredients may be used in the liquid or mouth rinse compositions of this invention, including those known and used in the art. Examples of such ingredients include thickeners, suspending agents and softeners. Thickeners and suspending agents useful in the compositions of the present invention can be found in U.S. Pat. 5,328,682 to Pullen et al.*.* In certain embodiments, these are incorporated in amounts of from about 0.1% w/v to about 0.6% w/v, or about 0.5% w/v of the composition.

In certain embodiments of the present invention the liquid for use in the present invention is a mouthwash liquid comprising water and one or more essential oils selected from the group consisting of menthol, thymol, eucalyptol, methyl salicylate and combinations of two or more thereof. In certain embodiments, the mouthwash liquid comprising water and all four essential oils menthol, thymol, eucalyptol, and methyl salicylate. In certain embodiments, the mouthwash liquid further comprises at least one surfactant. In certain embodiments the mouthwash liquid further comprises ethanol. In certain embodiments, the mouthwash liquid is free of ethanol.

In certain embodiments, more than one liquid may be used in the system and methods of the invention. For example, a cleaning solution may be applied to the oral cavity, followed by a second solution containing, for example, a whitening agent or an antimicrobial agent. Solutions also may include a plurality of agents to accomplish more than one benefit with a single application. For example, the solution may include both a cleansing agent and an agent for ameliorating a detrimental condition, as further discussed below. In addition, a single solution may be effective to provide more than one beneficial effect to the oral cavity. For example, the solution may include a single agent that both cleans the oral cavity and acts as an antimicrobial, or that both cleans the oral cavity and whitens teeth.

### Appliance

Any suitable article that is configured to be held in the mouth of a user with at least one nozzle in fluid communication with one or more surfaces of the user's oral cavity may be used as an appliance herein. In certain embodiments, when in use, the appliance functions to receive fluid from the fluid controller which is directed through at least one nozzle therein to a user's oral cavity.

In certain embodiments, the article comprises a first and second plurality of nozzles and is configured to be held in the mouth of a user with the first and second plurality of nozzles in fluid communication with one or more surfaces of the user's oral cavity. When in use, the appliance functions to receive fluid from the fluid controller through the first plurality of nozzles onto the surfaces of the oral cavity which may then be removed from the oral cavity through the second plurality of nozzles. The appliance may also be configured such that fluid may be directed from the fluid controller through the second plurality of nozzles onto the surfaces of the oral cavity and removed from the oral cavity through the first plurality of nozzles.

In certain embodiments, the appliance comprises a mouthpiece. Any suitable mouthpiece comprising at least one nozzle and configured to be held in the mouth to introduce fluid into the oral cavity in accord with this invention may be used. In certain embodiments, the mouthpiece comprises a first manifold in fluid communication with at least one nozzle such that fluid from the fluid controller can be directed into the first manifold and from the first manifold through at least one nozzle onto the surfaces of the oral cavity. In such embodiments, the mouthpiece may further comprise a first port in fluid communication with the first manifold through which fluid from the fluid controller is directed to the first manifold. Several mouthpiece embodiments will be described later in this disclosure.

In certain embodiments, the mouthpiece comprises a first and second manifold in fluid communication with a first and second plurality of nozzles, respectively, such that fluid from the fluid controller can be directed into the first manifold and from the first manifold through a first plurality of nozzles onto the surfaces of the oral cavity and/or fluid can be removed from the oral cavity through the first plurality of nozzles and through the first manifold. Likewise, fluid may be directed to the oral cavity from the fluid communicator through the second manifold and second plurality of nozzles and/or removed from the oral cavity through the second plurality of nozzles and then the second manifold. The mouthpiece may further comprise first and second ports in fluid communication with the first and second manifolds, respectively.

The mouthpiece may be comprised of multiple components. The mouthpiece comprises a chamber for maintaining the entrained fluid proximate the plurality of surfaces, i.e. fluid-contacting-chamber (FCC). By "proximate", it is meant that the fluid contacts the surfaces. The FCC is defined by the space bounded by the front inner wall and rear inner wall of the appliance, and a wall, or membrane, extending between and integral with the front and rear inner walls of the appliance, and in certain embodiments, an optional rear gum-sealing membrane. Together, the front and rear inner walls, and the wall extending there between form the FCC. The general shape of the FCC is that of a "U" or an "n", depending on the orientation of the appliance, which follows the teeth to provide uniform and optimized contact by the fluid. The FCC may be flexible or rigid depending on the particular appliance. The front and rear inner walls of the FCC each include a plurality of openings, or slots, or nozzles through which entrained fluid is directed to contact the plurality of surfaces of the oral cavity.

The FCC design may be optimized for maximum effectiveness as it relates to the size, shape, thickness, materials, volume created around the teeth/gingiva, nozzle design and placement as it relates to the oral cavity and the teeth in conjunction with the manifold and gingival margin seal to provide comfort and minimize the gagging reflex of the user. The combination of the above provides effective contact of the teeth and gingival area by the entrained fluid.

The FCC provides a controlled and isolated, or semi-isolated, environment, i.e. the FCC, to contact teeth and/or gingival area with entrained fluids, and preferably to remove spent fluids, as well as debris, plaque, etc., from the FCC. The FCC also allows increased flow rates and pressure of fluids without drowning the individual nozzles when significant flow rates are required to provide adequate cleaning, for example. The FCC also allows reduced fluid quantities and flow rates when required, as only the area within the FCC is being contacted with entrained fluid, not the entire oral cavity. The FCC also allows controlled delivery and duration of contact of entrained fluid on, through and around teeth and the gingival area, allowing increased concentrations of fluids on the area being contacted by the fluid, thereby providing more effective control and delivery of entrained fluid.

The device may incorporate a switching mechanism, which would allow it to be operable only when in the correct position in the mouth. In some embodiments, the appliance may include both upper and lower sections to provide substantially simultaneous contact of the plurality of surfaces of the oral cavity by entrained fluid.

The number and location of openings, also referred to herein as nozzles, contained within the inner walls of the appliance through which the entrained fluid is directed will vary and be determined based upon the circumstances and environment of use, the particular user and the beneficial effect being sought. The cross-sectional geometry of the openings may be circular, elliptical, trapezoidal, or any other geometry that provides effective contact of the surfaces of the oral cavity by the fluid. The location and number of openings may be designed to direct jets of entrained fluid in a variety of spray patterns effective for providing the desired beneficial effect. In certain embodiments, opening diameters may be from about 0.1 to about 3 mm, or from about 0.35 mm to about 0.8 mm, or about 0.5 mm, to provide effective cleaning and average jet velocities and coverage. The number of nozzles for simultaneous jet can be from 1 to 500, or 100-300. The horizontal spacing, or distance between the nozzles can be 0.5mm to 25mm, or 1mm to 10mm, or 3mm. Average jet velocities are about 0.5 m/s to about 100 m/s, or about 1 m/s to about 50 m/s. Nozzle coverage ranges from about 1mm² to about 20mm², or about 1mm² to about 10mm², or about 1mm² to about 5mm². The volume of each pulse from each nozzle is about 0.1microliter to about 5ml, or about 0.1microliter to about 1ml, or about 5 microliter to about 100 microliter.

Optimal opening placement and direction/angles allows coverage of substantially all teeth surfaces in the area if the oral cavity to be contacted by entrained fluid, including but not limited to interdental, top, side, back, and gingival pocket surfaces. In alternate embodiments, the openings could be of different sizes and different shapes to provide different cleaning, coverage and spray patterns, to adjust velocities, density and fan patterns (full cone, fan, partial, cone, jet), or due to formulation consideration. Nozzles could also be designed to be tubular and or extend from the FCC membrane to provide directed spray, or act as sprinkler like mechanism to provide extended coverage across the teeth, similar to a hose sprinkler system. The nozzles are preferably integral to the inner walls of the FCC membrane and can be incorporated into the inner walls through any number of assembly or forming techniques known in the art (insert molded, formed in membrane through machining, injection molding, etc.).

The FCC may be an elastomeric material such as ethylene vinyl acetate (EVA), thermoplastic elastomer (TPE), or silicone, to allow motion of the inner walls and provide a greater jet coverage area with minimal mechanics, reducing the volumetric flow requirements to achieve optimized performance, while providing a softer and more flexible material to protect the teeth if direct contact with the teeth is made. A flexible membrane may also provide acceptable fitment over a large range of users, due to its ability to conform to the teeth. Alternatively, the FCC could be made of a rigid or semi-rigid material, such as but not limited to a thermoplastic.

In an alternate embodiment, the FCC could also include abrasive elements such as filaments, textures, polishing elements, additives (silica, etc.), and other geometric elements that could be used for other cleaning and/or treatment requirements as well as ensuring minimal distance between the teeth and FCC for, but not limited to, treatment, cleaning, and positioning.

The FCC could be created via a variety of methods such as, but not limited to, machining, injection molding, blow molding, extrusion, compression molding, and/or vacuum forming. It can also be created in conjunction with the manifold, but incorporating the manifold circuitry within the FCC, and/or over-molded onto the manifold to provide a unitary construction with minimal assembly.

In one embodiment, the FCC may be fabricated separately and then assembled to the manifolds, utilizing any number of assembling and sealing techniques, including adhesives, epoxies, silicones, heat sealing, ultrasonic welding, and hot glue. The FCC is designed in a way that, when assembled with the manifold, it effectively and efficiently creates the preferred dual manifold design without any additional components.

In certain embodiments, the FCC can also be designed or used to create the gingival sealing area. In certain embodiments, a vacuum is applied within the FCC, which improves the engagement of the mouthpiece to form a positive seal with the gingival in the oral cavity. In other embodiments, a pressure is applied outside the FCC, within the oral cavity, which improves the engagement of the appliance to form a positive seal with the gingival in the oral cavity. In yet other embodiments, a denture-like adhesive may be applied around the mouthpiece during the initial use to provide a custom reusable resilient seal when inserted into the oral cavity for a particular user. It would then become resiliently rigid to both conform and provide a positive seal with the guns and on subsequent applications. In another embodiment, the seal could be applied and/or replaced or disposed of after each use.

The mouthpiece also comprises a first manifold for containing the entrained fluid and for providing the fluid to the FCC through the openings of the front inner wall, and a second manifold for containing the entrained fluid and for providing the fluid to the chamber through the openings of the rear inner wall. This design provides a number of different options, depending on what operation is being conducted. For instance, in a cleaning operation, it may be preferable to deliver jets of entrained fluid into the FCC directly onto the teeth from one side of the FCC from the first manifold and then evacuate/pull the fluid around the teeth from the other side of the FCC into the second manifold to provide controlled interdental, gum line and surface cleaning. This flow from the one side of the FCC could be repeated a number of times in a pulsing action before reversing the flow to deliver jets of entrained fluid from the second manifold and evacuating/pulling the fluid through the back side of the teeth into the first manifold for a period of time and/or number of cycles. Such fluid action creates a turbulent, repeatable and reversible flow, thus providing reciprocation of the fluid about the surfaces of the oral cavity. For example, the fluid controller may operate to alternately: (i) direct entrained fluid through the first plurality of nozzles of the appliance while simultaneously applying vacuum to the second plurality of nozzles to remove fluid from the appliance; and (ii) direct entrained fluid through the second plurality of nozzles of the appliance while simultaneously applying vacuum to the first plurality of nozzles to remove fluid from the appliance. The terms "reciprocating movement of fluid(s)" and "reciprocation of fluid(s)" are used interchangeably herein. As used herein, both terms mean alternating the direction of flow of the entrained fluid(s) back and forth over surfaces of the oral cavity of a mammal from a first flow direction to a second flow direction that is opposite the first flow direction.

In alternate embodiments, the manifold can be of single manifold design providing pushing and pulling of the entrained fluid through the same sets of jets simultaneously, or can be any number of manifold divisions to provide even greater control of the fluid delivery and removal of the cleaning and fluid treatment. In the multi-manifold also can be designed to have dedicated delivery and removal manifolds. The manifolds can also be designed to be integral to and/or within the FCC.

The material for the manifold would be a semi-rigid thermoplastic, which would provide the rigidity necessary not to collapse or burst during the controlled flow of the fluids, but to provide some flexibility when fitting within the user's mouth. To minimize fabrication complexity, number of components and tooling cost, the dual manifold is created when assembled with the FCC. The manifold could also be multi-component to provide a softer external "feel" to the teeth/gums utilizing a lower durometer elastomeric material, such as, but not limited to, a compatible thermoplastic elastomer (TPE). The manifold could be created via a variety of methods such as, but not limited to machining, injection molding, blow molding, compression molding, or vacuum forming.

As shown, the appliance also comprises a first port for conveying the fluid to and from the first manifold and a second port for conveying the fluid to and from the second manifold, and means for providing an effective seal of the directing means within the oral cavity, i.e. a gingival seal. In certain embodiments, the first and second ports may serve both to convey fluid to and from the first and second manifolds and to attach the appliance to the means for providing fluid to the appliance.

As noted above and described in more detail below, in certain embodiments, the system of the present invention is configured such that two sources of liquid and/or gas are combined to form entrained fluid between the fluid controller and the nozzles, e.g. before or within one or more of the manifolds and/or ports of the appliance. In certain of such embodiments, the appliance comprises a manifold and/or port configured (a) to receive a gas pulsed from the fluid controller directed to at least one nozzle and (b) to allow a separate liquid to be combined with the pulse within the manifold and/or port to form an entrained fluid directed to at least one nozzle.

### Fluid Controller

Any fluid controller suitable for directing fluid to at least one nozzle of an appliance in accord with the present invention may be used herein. In general, the fluid controller will comprise one or more portals, passages, and/or conduits, or the like, through which fluid may be received and conveyed to achieve the desired functions of the claimed invention, e.g. receiving fluid from a fluid source; and/or directing fluid to at least one nozzle of an appliance; and/or removing fluid from an appliance. In certain embodiments, the fluid controller may comprise, or otherwise be controlled by, a logic circuit and/or mechanically controlled circuit. In certain embodiments, the fluid controller may comprise, or otherwise be powered by, a power source, such as but not limited to, electricity provided through a plug, batteries, whether rechargeable or disposable, and the like.

In certain embodiments, the fluid controller comprises a reciprocating flow controller. In general the reciprocating flow controller is designed to alternate the flow of fluid to different portions of the appliance to allow for reciprocating flow of fluid across one or more surfaces of the oral cavity therein. For example, in certain embodiments, the reciprocating flow controller may have a first position which directs fluid to a first manifold and through at least a first nozzle of an appliance into the oral cavity in a first direction, and a second position which directs fluid to a second manifold and through at least a second nozzle of an appliance into the oral cavity in a second direction. The reciprocating flow controller may alternate between these two positions to alternate the flow of fluid in the appliance and thereby reciprocate fluid across the surfaces of the oral cavity within the appliance. Several reciprocating flow controller embodiments will be described later in this disclosure.

### Device/System

In certain embodiments, devices of the present invention may include a means for attaching or connecting the device to a reservoir for containing the liquid, gas, or the liquid/gas blend. The reservoir may be removably attached to the device. In this case, the reservoir and the device may comprise means for attaching one to the other. After completion of the process, the reservoir may be discarded and replaced with a different reservoir, or may be refilled and used again. In other embodiments, the device will include a reservoir integral with the device. In embodiments where the device may be attached to a base unit, as described herein, the reservoir, whether integral with the device or removably attached to the device, may be refilled from a supply reservoir which forms a part of the base unit. Where a base unit is utilized, the device and the base unit will comprise means for attaching one to the other.

The device will comprise a power source for powering one or more components of the device. The power source may be contained within the device, e.g. in the handle of the device, for example, batteries, whether rechargeable or disposable. Where a base unit is employed, the base may include the power source. In other embodiments, the base unit may include a charger for recharging batteries contained within the device. In another embodiment, compressed gas, such as in the form of a pressurized cartridge could be used as the power source for powering one or more components of the device. The cartridge could be disposable after one or more uses, or could be repressurized for subsequent uses.

In certain embodiments, the devices of the present invention include the appliance as a non-removable part of the device. In other embodiments, the appliance is removeably attached to the device via one or more connectors. In such embodiments, multiple users may attach and use their own separate appliances with the device.

As used herein, "means for conveying fluid" includes structures through which liquid, gas, or entrained fluid may travel or be transported throughout the systems according to the invention and includes, without limitation passages, conduits, tubes, ports, portals, channels, lumens, pipes and manifolds. Such means for conveying fluids may be utilized in devices for providing reciprocation of entrained fluids and means for directing entrained fluids onto and about surfaces of the oral cavity. Such conveying means also provide fluid to the directing means and provides gas, liquid, and/or entrained fluid to the reciprocation means from a reservoir for containing gas, liquid, or entrained fluid, whether the reservoir is contained within a hand-held device containing the reciprocation means or a base unit. The conveying means also provides liquid or gas from a base unit to reservoirs contained within the hand-held device. Described herein are methods, devices and systems useful in providing a beneficial effect to an oral cavity of a mammal, e.g. a human.

Methods entail contacting a plurality of surfaces of the oral cavity with a entrained fluid that is effective for providing the desired beneficial effect to the oral cavity. In such methods, reciprocation of the entrained fluid(s) over the plurality of surfaces of the oral cavity is provided under conditions effective to provide the desired beneficial effect to the oral cavity. Contact of the plurality of surfaces by the entrained fluid may be conducted substantially simultaneous. By substantially simultaneous, it is meant that, while not all of the plurality of surfaces of the oral cavity are necessarily contacted by the entrained fluid at the same time, the majority of the surfaces are contacted simultaneously, or within a short period of time to provide an overall effect similar to that as if all surfaces are contacted at the same time.

The conditions for providing the desired beneficial effect in the oral cavity may vary depending on the particular environment, circumstances and effect being sought. The different variables are interdependent in that they create a specific velocity of the entrained fluid. The velocity requirement may be a function of the formulation in some embodiments. For example, with change in the viscosity, additives, e.g. abrasives, shear thinning agents, etc., and general flow properties of the formulation, velocity requirements of the jets may change to produce the same level of efficacy. Factors which may be considered in order to provide the appropriate conditions for achieving the particular beneficial effect sought include, without limitation, the velocity and/or flow rate and/or pressure of the entrained fluid stream, pulsation of the entrained fluid, the spray geometry or spray pattern of the entrained fluid, the temperature of the entrained fluid and the frequency of the reciprocating cycle of the fluid. In some embodiments, the fluid flow rate (total all nozzles/simultaneous pulse) may be from about 0.1microliter to about 15ml, or about 0.1microliter to about 5ml.

Once having the benefit of this disclosure, one skilled in the art will recognize that the various factors may be controlled and selected, depending on the particular circumstances and desired benefit sought.

In addition to generally improving the oral hygiene of the oral cavity by cleaning, for example, removal or disruption of plaque build-up, food particles, biofilm, etc., the inventions are useful to ameliorate detrimental conditions within the oral cavity and to improve the cosmetic appearance of the oral cavity, for example whitening of the teeth. Detrimental conditions may include, without limitation, caries, gingivitis, inflammation, symptoms associated with periodontal disease, halitosis, sensitivity of the teeth and fungal infection. The liquids themselves may be in various forms, provided that they have the flow characteristics suitable for use in devices and methods of the present invention. For example, the liquids may be selected from the group consisting of solutions, emulsions and dispersions. In certain embodiments, the liquid may comprise a particulate, e.g. an abrasive, dispersed in a liquid phase, e.g. an aqueous phase. In such cases, the abrasive would be substantially homogeneously dispersed in the aqueous phase in order to be applied to the surfaces of the oral cavity. In other embodiments, an oil-in-water or water-in-oil emulsion may be used. In such cases, the liquid will comprise a discontinuous oil phase substantially homogeneously dispersed within a continuous aqueous phase, or a discontinuous aqueous phase substantially homogenously dispersed in a continuous oil phase, as the case may be. In still other embodiments, the liquid may be a solution whereby the agent is dissolved in a carrier, or where the carrier itself may be considered as the agent for providing the desired beneficial effect, e.g., an alcohol or alcohol/water mixture, usually having other agents dissolved therein.

Disclosed herein are systems, e.g. systems comprising oral care devices, for example a dental cleaning apparatus, suitable for in-home use and adapted to direct entrained fluid pulses onto a plurality of surfaces of a tooth and/or the gingival area, as well as methods utilizing such systems. In certain embodiments the surfaces of the oral cavity are contacted by the fluid substantially simultaneously. As used herein, reference to the gingival area includes, without limitation, reference to the sub-gingival pocket. The appropriate entrained fluid is directed onto a plurality of surfaces of teeth and/or gingival area substantially simultaneously in a reciprocating action under conditions effective to provide cleaning, and/or general improvement of the cosmetic appearance of the oral cavity and/or amelioration of a detrimental condition of the teeth and/or gingival area, thereby providing generally improved oral hygiene of teeth and/or gingival area. For example, one such device cleans teeth and/or the gingival area and removes plaque using an appropriate cleaning entrained fluid by reciprocating the entrained fluid back and forth over the front and back surfaces and inter-proximal areas of the teeth, thereby creating a cleaning cycle while minimizing the amount of cleaning fluid used.

Systems of the invention comprise devices that provide reciprocation of the entrained fluid pulses, which devices comprise a means for controlling reciprocation of the fluid. The controlling means includes means for conveying the entrained fluid to and from a means for directing the entrained fluid onto the plurality of surfaces of the oral cavity. In certain embodiments, the means for providing reciprocation of the entrained fluid comprises a plurality of portals for receiving and/or discharging the fluid, a plurality of passages, or conduits, through which the fluid is conveyed, and means for changing the direction of flow of the fluid to provide reciprocation of the fluid. The controlling means may be controlled by a logic circuit and/or a mechanically controlled circuit.

In certain embodiments, devices for providing reciprocation may include a means for attaching or connecting the device to a reservoir for containing the liquid, gas, or the liquid/gas blend. The reservoir may be removably attached to the device. In this case, the reservoir and the device may comprise means for attaching one to the other. After completion of the process, the reservoir may be discarded and replaced with a different reservoir, or may be refilled and used again. In other embodiments, the reciprocating device will include a reservoir integral with the device. In embodiments where the device may be attached to a base unit, as described herein, the reservoir, whether integral with the device or removably attached to the device, may be refilled from a supply reservoir which forms a part of the base unit. Where a base unit is utilized, the device and the base unit will comprise means for attaching one to the other.

The device will comprise a power source for driving the means for reciprocating entrained fluids. The power source may be contained within the device, e.g. in the handle of the device, for example, batteries, whether rechargeable or disposable. Where a base unit is employed, the base may include means for providing power to the device. In other embodiments, the base unit may include means for recharging the rechargeable batteries contained within the device. In another embodiment, compressed gas, such as in the form of a pressurized cartridge could be used as the power source for powering one or more components of the device. The cartridge could be disposable after one or more uses, or could be repressurized for subsequent uses.

Devices for providing reciprocation of entrained fluids will include means for attaching the device to means for directing the entrained fluid onto the plurality of surfaces of the oral cavity, e.g. an appliance such as an applicator, tray or mouthpiece. In certain embodiments, the directing means provides substantially simultaneous contact of the plurality of surfaces of the oral cavity by the fluid. The attachment means may provide removable attachment of the applicator to the device. In such embodiments, multiple users may use their own appliances with the single device comprising the reciprocating means. In other embodiments, the attachment means may provide a non-removable attachment to the appliance, whereby the appliance is an integral part of the device. Devices for providing reciprocation as described above may be contained within a housing also containing other device components so as to provide a hand-held device suitable for providing entrained fluids to the directing means, as described herein below.

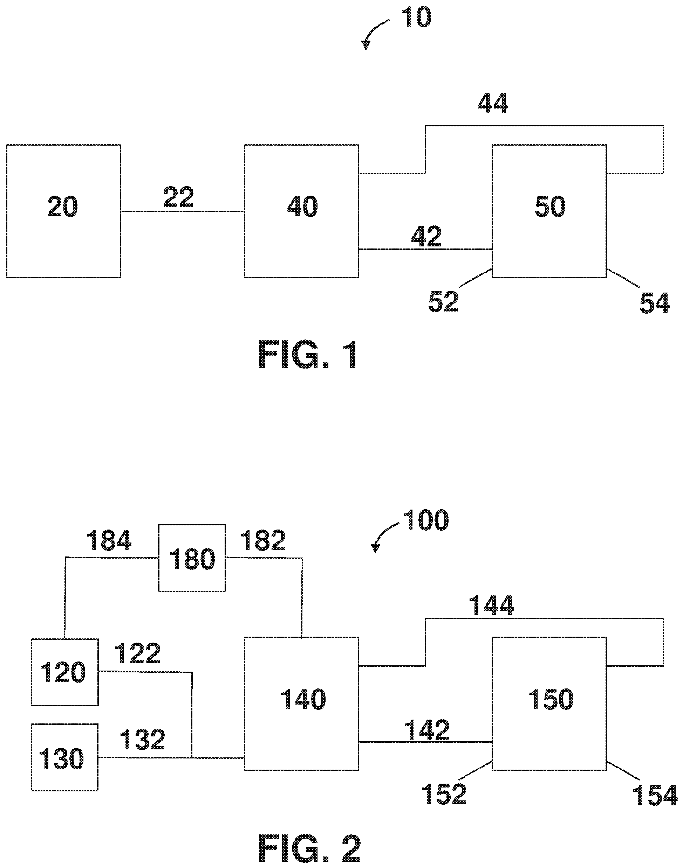

FIG. 1 is a schematic drawing of a first embodiment of a system according to the present invention. The figure shows system **10,** with components including: fluid supply reservoir **20,** reciprocating flow controller **40,** means for directing the fluid onto the plurality of surfaces of the oral cavity (appliance **50**), and tubes **22, 42,** and **44** for conveying the fluid throughout the system.

Tube **22** conveys fluid from reservoir **20** to reciprocating flow controller **40.** Tubes **42** and **44** convey fluid from reciprocating flow controller **40** to appliance **50.** Tube **42** conveys fluid to the first side **52** of appliance **50,** while tube **44** conveys fluid to the second side **54** of appliance **50.**

In this embodiment, fluid supply reservoir **20** contains entrained fluid. The fluid may be under sufficient pressure to be delivered to reciprocating flow controller **40,** and further to appliance **50.** In other embodiments, a pump, such a piston pump or rotary pump, may be located between fluid supply reservoir **20** and reciprocating flow controller **40** to supply sufficient pressure to delivered fluid to reciprocating flow controller **40,** and further to appliance **50.**

Fluid supply reservoir **20** may be made of glass, plastic or metal. Fluid supply reservoir **20** may be integral to system **10** and refillable. In some embodiments, fluid supply reservoir **20** may be a replaceable fluid supply, such as a single or multi-use cartridge, detachably connected to system **10.**

In some embodiments, fluid supply reservoir **20** and/or tubes **22, 42,** and **44** may include a heat source to pre-warm the fluid prior to direction into appliance **50** for application to the surfaces of the oral cavity. The temperature should be maintained within a range effective to provide efficacy and comfort to the user during use.

Appliance **50,** discussed in detail herein below, could be integral with, or detachably connected to, reciprocating flow controller **40** by way of tubes **42, 44** and further attachment means (not shown). It could be have internally, easily cleanable filters for trapping food particles. When positioned within the oral cavity, e.g. about the teeth and gums, appliance **50** may form an effective fit or seal against the gums, and includes means to direct fluid against surfaces of the oral cavity, e.g. surfaces of the teeth.

Entrained fluid in fluid supply reservoir **20** flows through tube **22** to reciprocating flow controller **40.** There may be a one-way flow valve in tube **22** to prevent back-pressure from allowing fluid flow from controller **40** back to reservoir **20.** Entrained fluid flows from reciprocating flow controller **40** to appliance **50** either through tube **42** or **44,** depending on the flow direction setting of flow controller **40.**

In the cleaning operation, system **10** produce a series of entrained fluid pulses in appliance **50.** In some embodiments flow controller **40** creates the series of entrained fluid pulses. In other embodiments, entrained fluid pulses are generated by a controller located in any of tubes **22, 42,** or **44.**

The actions of system **10** may be controlled by a logic circuit, which may include a program to start the reciprocation cycle, a program to execute the reciprocation cycle, i.e. to cause fluid to be reciprocated about the teeth, thereby providing the beneficial effect to the oral cavity, e.g. cleaning the teeth, a program to empty appliance **50** at the end of the reciprocation cycle, and a self-cleaning cycle to clean the system between uses, or at pre-set or automatic cleaning times.

Though not shown, a face panel with a series of switches and indicator lights may also be incorporated into system **10.** Switches may include, but are not limited to, on/off, run the reciprocation program, empty system **10,** and clean system **10.** Indicator, or display, lights include, but are not limited to, power on, charging, reciprocation program running, system emptying, cleaning results or feedback, and self-cleaning cycle in operation. In embodiments where fluid is pre-warmed prior to direction into appliance **50,** a display light could be used to indicate that the fluid is at the proper temperature for use.

One method of using system **10** to clean teeth is as follows. In the first step, the user positions appliance **50** in the oral cavity about the teeth and gingival area. In use of the system according to the invention, the user pushes a start button initiating the cleaning process. The cleaning process is as follows:
1. System **10** is activated to begin dispensing entrained fluid pulses from reservoir **20** to appliance **50** via tube **22,** reciprocating flow controller **40,** and tube **42.** Entrained fluid is used to clean the teeth and gingival area from first side **52** of appliance **50.**
2. Reciprocating flow controller **40** is then activated to change the fluid flow from tube **42** to tube **44.** The pulses of entrained fluid is used to clean the teeth and gingival area from second side **54** of appliance **50.**
3. To reciprocate the cleaning fluid, steps 1 and 2 are repeated as the entrained fluid pulses are used to clean the teeth and gingival area from first side **52,** and then second side **54** of appliance **50,** respectively.
4. The reciprocation cycle as described continues until the time required for cleaning has expired, or the desired numbers of cycles are complete.

It is noted that there may be a delay between steps 1 and 2 (in either or both, directions), allowing a dwell time where the fluid is allowed to contact the teeth without flow.

In this embodiment, entrained fluid, once it contacts the teeth and gingival surface, disperse into the oral cavity. In other embodiment, the liquid portion of the entrained fluid may be directed via tubes back to the reservoir **20** to be reused in the current, or a future cleaning process, or directed to disposal.

FIG. 2 is a schematic drawing of a second embodiment of a system according to the present invention. The figure shows system **100,** with components including: liquid supply reservoir **120,** gas supply reservoir **130,** reciprocating flow controller **140,** appliance **150,** vacuum pump **180,** and tubes **122, 132, 142, 144, 182,** and **184,** for conveying the fluid throughout the system.

Tube **132** conveys gas from gas reservoir **130** to reciprocating flow controller **140.** Tube **122** conveys liquid from liquid reservoir **120** to tube **132.** Tubes **142** and **144** convey entrained fluid from reciprocating flow controller **140** to appliance **150.** Tube **142** conveys fluid to the first side **152** of appliance **150,** while tube **144** conveys fluid to the second side **152** of appliance **150.**

In this embodiment, entrained fluid is created at the intersection of tubes **122** and **132,** prior to reciprocating flow controller **140.** Gas reservoir **130** may be under sufficient pressure to be delivered entrained fluid to reciprocating flow controller **140,** and further to appliance **150.** In some embodiments, liquid reservoir **120** may be under sufficient pressure, such as head pressure, to deliver liquid to the point of intersection of tubes **122** and **132.** In other embodiments, a pump may be located between liquid reservoir **120** and the intersection of tubes **122** and **132** to delivered liquid to the point of intersection of tubes **122** and **132.**

In some embodiments, the Venturi effect is used to entrain the liquid in the gas stream. The Venturi effect is the creation of a partial vacuum when the flow of a fluid is restricted, increasing its speed of flow. In this embodiment, gas tube **132** is narrowed at one point before widening again. Liquid tube **122** is attached to the narrowed portion of gas tube **132,** and liquid in liquid tube **122** is drawn into and entrained in gas in gas tube **132.**

In this embodiment, entrained fluid, once it contacts the teeth and gingival surface, separates into a gas phase and a liquid phase. The gas phase mainly disperses into the oral cavity, while the liquid phase is partially, or fully, recaptured. Tube **182** connects vacuum pump **180** to reciprocating flow controller **140.** Pump **180** creates a negative pressure that draws the liquid from appliance **150** back through reciprocating flow controller **140** and delivers it back to liquid reservoir **120** via tube **184.**

Liquid in liquid supply reservoir **120** flows through tube **122** to the point of intersection of tubes **122** and **132** and on to reciprocating flow controller **140.** There may be a one-way flow valve in tube **122** and/or tube **132** to prevent back-pressure from allowing liquid flow back to reservoir **120,** or gas to flow back to reservoir **130,** respectively. After entrainment, entrained fluid flows to reciprocating flow controller **140,** and then from reciprocating flow controller **140** to appliance **150** either through tube **142** or **144,** depending on the flow direction setting of flow controller **140.**

In the cleaning operation, system **100** produce a series of entrained fluid pulses in appliance **150.** In some embodiments flow controller **140** creates the series of entrained fluid pulses. In other embodiments, entrained fluid pulses are generated by a controller located in any of tubes **122, 132, 142,** or **144.**

One method of using system **100** to clean teeth is as follows. In the first step, the user positions appliance **150** in the oral cavity about the teeth and gingival area. In use of the system according to the invention, the user pushes a start button initiating the cleaning process. The cleaning process is as follows:
1. System **100** is activated to begin dispensing liquid from liquid reservoir **120** and gas from gas reservoir **130** to the point of intersection of tubes **122** and **132,** and on to reciprocating flow controller **140** and then appliance **150** via tube **142,** reciprocating. Entrained fluid pulses are used to clean the teeth and gingival area from first side **152** of appliance **150.** The entrained fluid separates into gas and liquid phases.
2. The liquid returns to reciprocating flow controller **140** via tube **144,** flows to pump **180** via tube **182,** and is returned to liquid reservoir **120** via tube **184.**
3. Reciprocating flow controller **140** is then activated to change the fluid flow from tube **142** to tube **144.** Entrained fluid pulses are used to clean the teeth and gingival area from second side **154** of appliance **150.**
4. The liquid returns to reciprocating flow controller **140** via tube **142,** flows to pump **180** via tube **182,** and is returned to liquid reservoir **120** via tube **184.**
5. To reciprocate the cleaning fluid, steps 1 through 4 are repeated as the entrained fluid pulses are used to clean the teeth and gingival area from first side **152,** and then second side **154** of appliance **150,** respectively.
6. The reciprocation cycle as described continues until the time required for cleaning has expired, or the desired numbers of cycles are complete.

In this embodiment, the liquid portion of the entrained fluid is directed via tube **182** back to the liquid reservoir **120** to be reused in the current, or a future cleaning process. In other embodiments, pump **180** may direct the spent liquid to disposal.

In some embodiments, entrained fluid may be delivered from reciprocating flow controller **140** to first side **152** of appliance **150** while simultaneously being drawn by vacuum pump **180** from second side **154** of appliance **150.** When reciprocating flow controller **140** is activated to change the fluid flow from tube **142** to tube **144,** entrained fluid may be delivered from reciprocating flow controller **140** to second side **154** of appliance **150** while simultaneously being drawn by vacuum pump **180** from first side **152** of appliance **150.**

It is important to note that although **180** is termed "vacuum pump", **180** could also be used for other devices or methods for creating a pressure drop from reciprocating flow controller **140** to liquid reservoir **120.** Included are devices that produce the Venturi effect or temperature drops.

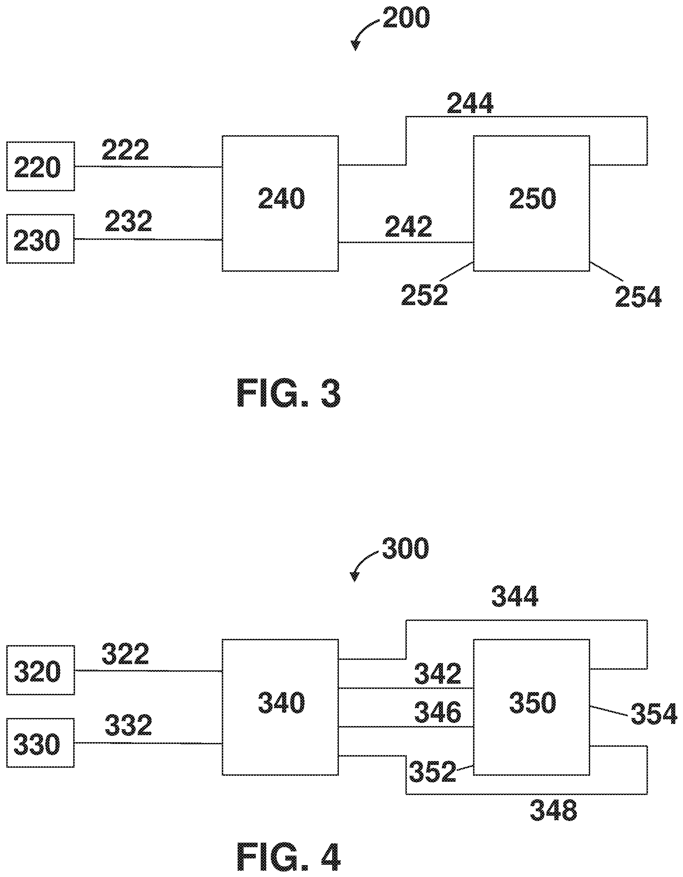

FIG. 3 is a schematic drawing of a third embodiment of a system according to the present invention. The figure shows system **200,** with components including: liquid supply reservoir **220,** gas supply reservoir **230,** reciprocating flow controller **240,** appliance **250,** and tubes **222, 232, 242,** and **244,** for conveying the fluid throughout the system.

Tube **222** conveys liquid from liquid reservoir **220** to reciprocating flow controller **240.** Tube **232** conveys gas from gas reservoir **230** to reciprocating flow controller **240.** Tubes **242** and **244** convey entrained fluid from reciprocating flow controller **240** to appliance **250.** Tube **242** conveys fluid to the first side **252** of appliance **250,** while tube **244** conveys fluid to the second side **25** of appliance **250.**

In this embodiment, entrained fluid is created in reciprocating flow controller **240.** Gas reservoir **230** may be under sufficient pressure to be delivered entrained fluid to reciprocating flow controller **240,** and further to appliance **250.** In some embodiments, liquid reservoir **220** may be under sufficient pressure, such as head pressure, to deliver liquid to reciprocating flow controller **240.** In other embodiments, a pump may be located between liquid reservoir **220** and reciprocating flow controller **240.**

In this embodiment, entrained fluid, once it contacts the teeth and gingival surface, disperse into the oral cavity. In other embodiment, some of the liquid portion of the entrained fluid may be directed via tubes back to the reservoir **220** to be reused in the current, or a future cleaning process, or directed to disposal.

Liquid in liquid supply reservoir **220** flows through tube **222** to reciprocating flow controller **240.** Gas in gas reservoir **230** flows through tube **232** to reciprocating flow controller **240.** There may be a one-way flow valve in tube **222** and/or tube **232** to prevent back-pressure from allowing liquid flow back to reservoir **220,** or gas to flow back to reservoir **230,** respectively.

After entrainment, entrained fluid flows to appliance **250** either through tube **242** or **244,** depending on the flow direction setting of flow controller **240.**

In the cleaning operation, system **200** produce a series of entrained fluid pulses in appliance **250.** In some embodiments flow controller **240** creates the series of entrained fluid pulses. In other embodiments, entrained fluid pulses are generated by a controller located in any of tubes **222, 232, 242,** or **244.**

One method of using system **200** to clean teeth is as follows. In the first step, the user positions appliance **250** in the oral cavity about the teeth and gingival area. In use of the system according to the invention, the user pushes a start button initiating the cleaning process. The cleaning process is as follows:
1. System **200** is activated to begin dispensing liquid from liquid reservoir **220** to reciprocating flow controller **240** via tube **222,** and gas from gas reservoir **230** to reciprocating flow controller **240** via tube **232.** Reciprocating flow controller **240** sends entrained fluid to first side **252** of appliance **250** via tube **242.** Entrained fluid pulses are used to clean the teeth and gingival area from first side **252** of appliance **250.** The entrained fluid separates into gas and liquid phases.
2. Reciprocating flow controller **240** is then activated to change the fluid flow from tube **242** to tube **244.** Entrained fluid pulses are used to clean the teeth and gingival area from second side **254** of appliance **250.** The entrained fluid separates into gas and liquid phases.
3. To reciprocate the cleaning fluid, steps 1 and 2 are repeated as the entrained fluid pulses are used to clean the teeth and gingival area from first side **252,** and then second side **254** of appliance **250,** respectively.
4. The reciprocation cycle as described continues until the time required for cleaning has expired, or the desired numbers of cycles are complete.

In this embodiment, entrained fluid, once it contacts the teeth and gingival surface, disperse into the oral cavity. In other embodiment, the liquid portion of the entrained fluid may be directed via tubes back to the reservoir **220** to be reused in the current, or a future cleaning process, or directed to disposal.

FIG. 4 is a schematic drawing of a fourth embodiment of a system according to the present invention. The figure shows system **300,** with components including: liquid supply reservoir **320,** gas supply reservoir **330,** reciprocating flow controller **340,** appliance **350,** and tubes **322, 332, 342, 344, 346,** and **348** for conveying the fluid throughout the system.

Tube **322** conveys liquid from liquid reservoir **320** to reciprocating flow controller **340.** Tube **332** conveys gas from gas reservoir **330** to reciprocating flow controller **340.** Tubes **342** and **344** convey liquid from reciprocating flow controller **340** to appliance **350.** Tube **342** conveys fluid to the first side **352** of appliance **350,** while tube **344** conveys fluid to the second side **35** of appliance **350.** Tubes **346** and **348** convey gas from reciprocating flow controller **340** to appliance **350.** Tube **346** conveys gas to the first side **352** of appliance **350,** while tube **348** conveys gas to the second side **354** of appliance **350.**

In this embodiment, entrained fluid is created in appliance **350.** Gas reservoir **330** may be under sufficient pressure to be delivered entrained fluid to appliance **350.** In some embodiments, liquid reservoir **320** may be under sufficient pressure, such as head pressure, to deliver liquid to appliance **350.** In other embodiments, a pump may be located between liquid reservoir **320** and reciprocating flow controller **340** to delivered liquid to appliance **350.**

In this embodiment, entrained fluid, once it contacts the teeth and gingival surface, disperse into the oral cavity. In other embodiment, some of the liquid portion of the entrained fluid may be directed via tubes back to the reservoir **320** to be reused in the current, or a future cleaning process, or directed to disposal.

Liquid in liquid supply reservoir **320** flows through tube **322** to reciprocating flow controller **340.** Gas in gas reservoir **330** flows through tube **332** to reciprocating flow controller **340.** There may be a one-way flow valve in tube **322** and/or in tube **332** to prevent back-pressure from allowing liquid flow back to reservoir **320,** or gas to flow back to reservoir **330,** respectively.

In the cleaning operation, system **300** produce a series of entrained fluid pulses in appliance **350.** In some embodiments flow controller **340** creates the series of entrained fluid pulses. In other embodiments, entrained fluid pulses are generated by a controller located in any of tubes **322, 332, 342, 344, 346,** and **348.**

One method of using system **300** to clean teeth is as follows. In the first step, the user positions appliance **350** in the oral cavity about the teeth and gingival area. In use of the system according to the invention, the user pushes a start button initiating the cleaning process. The cleaning process is as follows:
1. System **300** is activated to begin dispensing liquid from liquid reservoir **320** to reciprocating flow controller **340** via tube **322,** and gas from gas reservoir **330** to reciprocating flow controller **340** via tube **332.** Reciprocating flow controller **340** sends liquid to first side **352** of appliance **350** via tube **342,** and gas to first side **352** of appliance **350** via tube **346.** Entrained fluid is created in appliance **350.** The entrained fluid pulses are used to clean the teeth and gingival area from first side **352** of appliance **350.** The entrained fluid separates into gas and liquid phases.
2. Reciprocating flow controller **340** is then activated to change the fluid flow from tubes **342** and **346** to tubes **344** and **348.** Reciprocating flow controller **340** sends liquid to second side **354** of appliance **350** via tube **344,** and gas to second side **354** of appliance **350** via tube **348.** Entrained fluid pulses are used to clean the teeth and gingival area from second side **354** of appliance **350.** The entrained fluid separates into gas and liquid phases.
3. To reciprocate the cleaning fluid, steps 1 and 2 are repeated as the entrained fluid pulses are used to clean the teeth and gingival area from first side **352,** and then second side **354** of appliance **350,** respectively.
4. The reciprocation cycle as described continues until the time required for cleaning has expired, or the desired numbers of cycles are complete.

In this embodiment, entrained fluid, once it contacts the teeth and gingival surface, disperse into the oral cavity. In other embodiment, the liquid portion of the entrained fluid may be directed via tubes back to the reservoir **320** to be reused in the current, or a future cleaning process, or directed to disposal.

As mentioned in this embodiment, entrained fluid is created in appliance **350,** in other embodiments, tube **342** may intersect tube **346** prior to appliance **350,** creating entrained fluid prior to appliance **350.** In a similar fashion, and tube **344** may intersect tube **348** prior to appliance **350,** creating entrained fluid prior to appliance **350.**

Each embodiment described in FIG. 1, FIG. 2, FIG. 3, and FIG. 4 includes a reciprocating flow controller (**40, 140, 240,** and **340** in FIG. 1, FIG. 2, FIG. 3, FIG. 4, respectively).

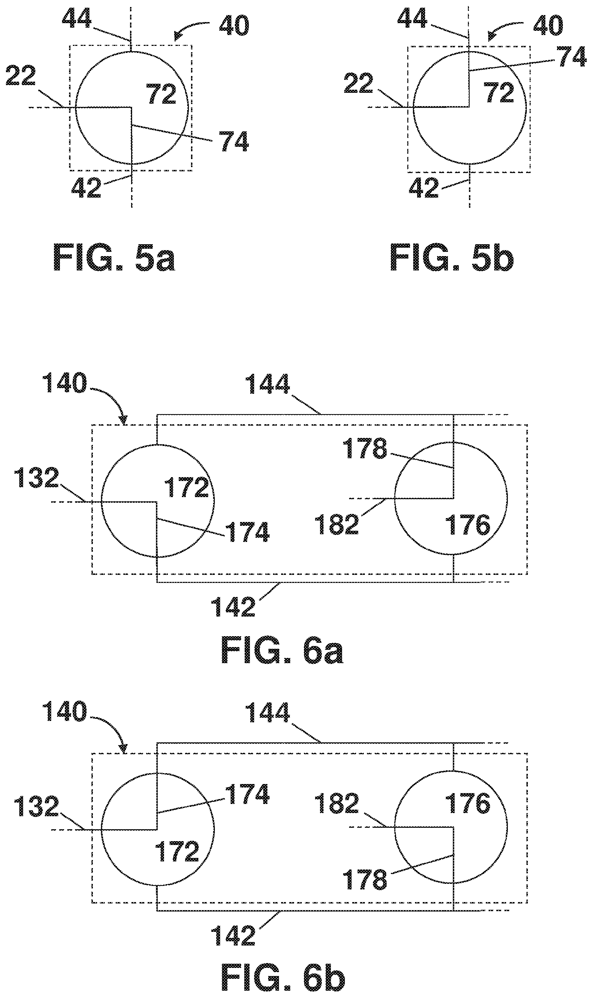

FIGs. 5a and 5b are schematic drawings of an embodiment of a reciprocating flow controller **40** used in first embodiment system according to the present invention. In FIG. 5a, reciprocating flow controller **40** is in a first position, while in FIG. 5b, reciprocating flow controller **40** is in a second position. In this embodiment, reciprocating flow controller **40** is in the form of an l-port valve **72.** An l-port valve is a ball valve which uses a hollow, perforated and pivoting ball with L-shaped hole **74** through the ball to control flow through the valve. It is open when the ball's hole is in line with the flow and closed when it is not in line with the flow. An l-port valve is pivoted 90-degrees to connect the center port to either side port.

In FIG. 5a, valve **72** is set so that entrained fluid from tube **22** passes through hole **74** and into tube **42.** To reciprocate fluid in system **10,** valve **72** is pivoted 90-degrees (as shown in FIG. 5b) so that fluid from tube **22** passes through hole **74** and into tube **44.**

FIGs. 6a and 6b are schematic drawings of an embodiment of a reciprocating flow controller **140** used in second embodiment system according to the present invention. In FIG. 6a, reciprocating flow controller **140** is in a first position, while in FIG. 6b, reciprocating flow controller **140** is in a second position. In this embodiment, reciprocating flow controller **140** is in the form of two l-port valves, **172** and **176.** Valve **172** has L-shaped hole **174** and valve **176** has L-shaped hole **178.**

In FIG. 6a, valve **172** is set so that entrained fluid from tube **132** passes through hole **174** and into tube **142.** Valve **176** is set so that liquid returning from second side **154** of appliance **150** via tube **144** passes through hole **178** and into tube **182.** To reciprocate fluid in system **100,** valves **172** and **176** are pivoted 90-degrees (as shown in FIG. 6b) so that entrained fluid from tube **132** passes through hole **174** and into tube **144,** while liquid returning from second side **154** of appliance **150** via tube **144** passes through hole **178** and into tube **182.**

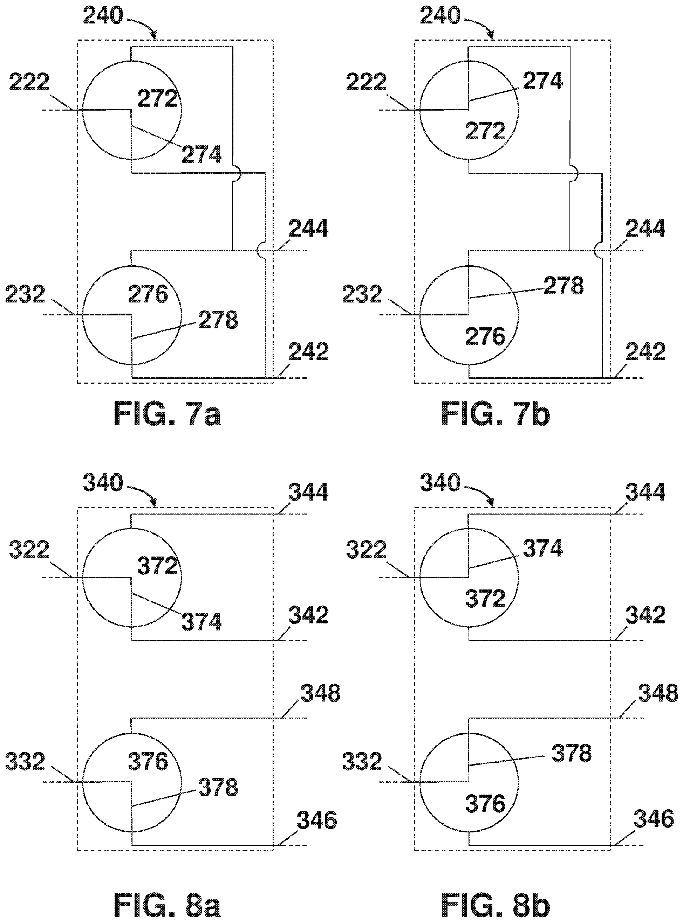

FIGs. 7a and 7b are schematic drawings of an embodiment of a reciprocating flow controller **240** used in third embodiment system according to the present invention. In FIG. 7a, reciprocating flow controller **240** is in a first position, while in FIG. 7b, reciprocating flow controller **240** is in a second position. In this embodiment, reciprocating flow controller **240** is in the form of two l-port valves, **272** and **276.** Valve **272** has L-shaped hole **274** and valve **276** has L-shaped hole **278.**

In FIG. 7a, valve **276** is set so gas from tube **232** passes through hole **278** and into tube **242.** Valve **272** is set so that liquid from tube **222** passes through hole **274** and intersects tube **242** at the point where entrainment occurs. As mentioned earlier, the Venturi effect may be used to entrain the liquid in the gas stream. In this embodiment, tube **242** is narrowed at one point before widening again. Liquid tube **222** is attached to the narrowed portion of tube **242,** and liquid in liquid tube **222** is drawn into and entrained in gas in tube **242.**

To reciprocate fluid in system **200,** valves **272** and **276** are pivoted 90-degrees (as shown in FIG. 7b) so that gas from tube **232** passes through hole **274** and into tube **244.** Valve **272** is set so that liquid from tube **222** passes through hole **274** and intersects tube **244** at the point where entrainment occurs. The Venturi effect may be used here to for entraining the liquid in the gas stream. Here, tube **244** is narrowed at one point before widening again. Liquid tube **222** is attached to the narrowed portion of tube **244,** and liquid in liquid tube **222** is drawn into and entrained in gas in tube **244.**

FIGs. 8a and 8b are schematic drawings of an embodiment of a reciprocating flow controller **340** used in fourth embodiment system according to the present invention. In FIG. 8a, reciprocating flow controller **340** is in a first position, while in FIG. 8b, reciprocating flow controller **340** is in a second position. In this embodiment, reciprocating flow controller **340** is in the form of two l-port valves, **372** and **376.** Valve **372** has L-shaped hole **374** and valve **376** has L-shaped hole **378.**

In FIG. 8a, valve **372** is set so liquid from tube **322** passes through hole **374** and into tube **342.** Valve **376** is set so that gas from tube **322** passes through hole **378** and into tube **346.** To reciprocate fluid in system **300,** valves **372** and **376** are pivoted 90-degrees (as shown in FIG. 8b) so that liquid from tube **322** passes through hole **374** and into tube **344.** Valve **376** is set so that gas from tube **332** passes through hole **378** and into tube **348.**

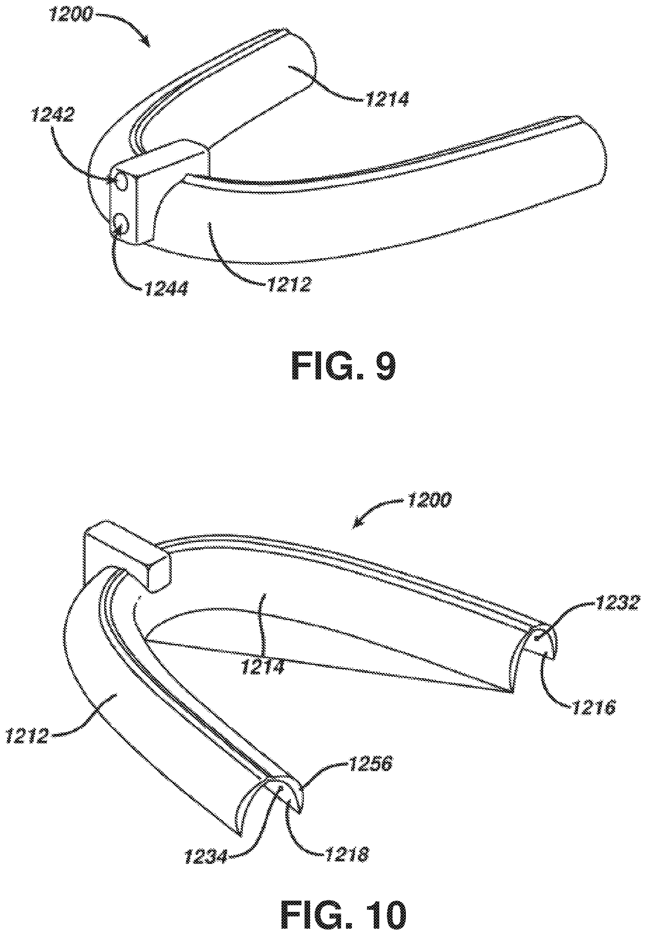

As mentioned earlier, appliance (**50, 150, 250** or **350**) may be in the form of an application tray, or mouthpiece. FIGS. 9 to 12 depict an embodiment of an application tray **1200** in which only the user's top or bottom teeth and gingival area are contacted with fluid. It should be understood that in other embodiments, application tray **1200** may be designed to substantially simultaneously contact both the top and bottom teeth and gingival area of the user, as depicted elsewhere herein.

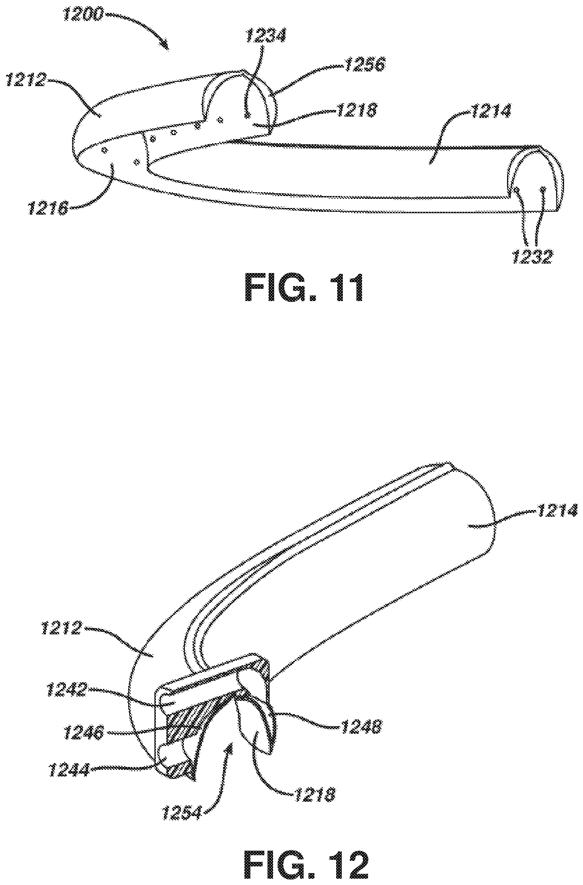

FIG. 9 is a top front perspective view of a application tray **1200** for use in accordance with the present invention. FIG. 10 is a top back view of the embodiment of the application tray **1200** of FIG. 9, while FIG. 11 is a bottom back view of the application tray **1200** of FIG. 9. The figures show application tray **1200** with outer front wall **1212,** outer back wall **1214,** inner front wall **1216,** and inner back wall **1218.** Inner front wall jet slots **1232** are located on inner front wall **1216,** while inner back wall jet slots **1234** are located on inner back wall **1218.** First port **1244** and second port **1242** enter application tray **1200** through outer front wall **1212.**

The number and location of inner front wall jet slot **1232** and inner back wall jet slot **1234** as shown in FIGS. 9 to 12 is exemplary and is not intended to limit the scope of the application tray. The actual number, shape and size of inner front wall jet slots **1232** and inner back wall jet slots **1234** affect the cleaning of the teeth and gums, and can be selected or designed to direct jets of cleaning fluid in a variety of spray patterns. The inner front wall jet slots **1232** and inner back wall jet slots **1234** shown in FIGS. 9 to 11 are only one embodiment of jet slot configuration.

In other embodiments, here may also be additional wall jet slots located on outer front wall **1212,** outer back wall **1214,** or both walls. These jets would allow entrained fluid to be directed to the oral cavity, or the inner lips, or the tongue, to promote cleaning or treating of these areas in the mouth.

FIG. 12 is a vertical sectional view of the application tray **1200** of FIG. 9. The figures show first manifold **1246,** defined as the space bordered by outer front wall **1212** and inner front wall **1216.** Second manifold **1248** is defined as the space bordered by outer back wall **1214** and inner back wall **1218.** The fluid contact chamber (FCC) **1254** is defined by inner front wall **1216,** inner back wall **1218** and inner base wall **1250.**

In one embodiment of a cleaning operation, entrained fluid enters first manifold **1246** through first port **1244** by pressure and then enters FCC **1254** through inner front wall jet slots **1232.** In this embodiment, jets of entrained fluid are first directed onto the front side of the teeth, gums, and/or gingival areas. Next, the flow in the manifolds is reversed. Entrained fluid enters second manifold **1248** through second port **1242** by pressure and then enters FCC **1254** through inner back wall jet slots **1234.** In the second portion of this embodiment, jets of entrained fluid are directed onto the back side of the teeth, gums, and/or gingival areas. The alternating of pressure through a number of cycles creates a turbulent, repeatable and reversible flow, thereby providing reciprocation of entrained over and about the surfaces of the oral cavity.

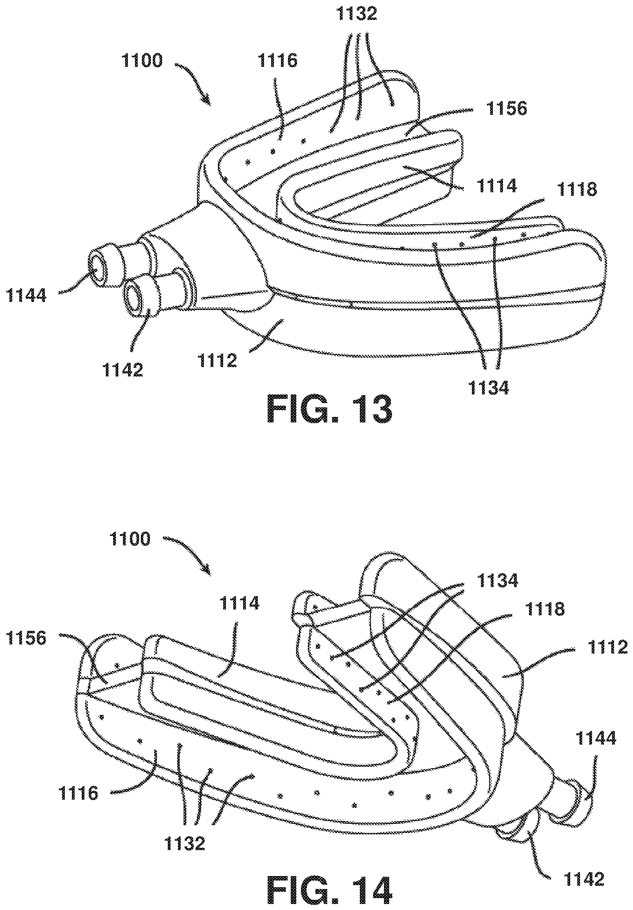

FIG. 13 is a top perspective view of a second embodiment of means for directing entrained fluid onto a plurality of surfaces in the oral cavity, e.g. an application tray **1100,** used with devices according to the present invention. FIG. 14 is a bottom perspective view of the application tray **1100** of FIG. 13. The figures show application tray **1100** with outer front wall **1112,** outer back wall **1114,** inner front wall **1116,** inner back wall **1118,** and base membrane, e.g. bite plate, **1156.** Inner front wall jet slots **1132** are located on inner front wall **1116,** while inner back wall jet slots **1134** are located on inner back wall **1118.** The inner front wall jet slots **1132** and inner back wall jet slots **1134** shown in FIGS. 13 and 14 are only one embodiment of jet slot configuration. First port **1142** and second port **1144** enter application tray **1100** through outer front wall **1112.**

In other embodiments, here may also be additional wall jet slots located on outer front wall **1112,** outer back wall **1114,** or both walls. These jets would allow entrained fluid to be directed to the oral cavity, or the inner lips, or the tongue, to promote cleaning or treating of these areas in the mouth.

FIGS. 13 and 14 depict an embodiment of an application tray **1100** in which the user's top and bottom teeth and/or gingival area are substantially simultaneously contacted with fluid to provide the desired beneficial effect. It should be understood that in other embodiments, application tray **1100** may be designed to clean and/or treat only the top or bottom teeth and/or gingival area of the user.

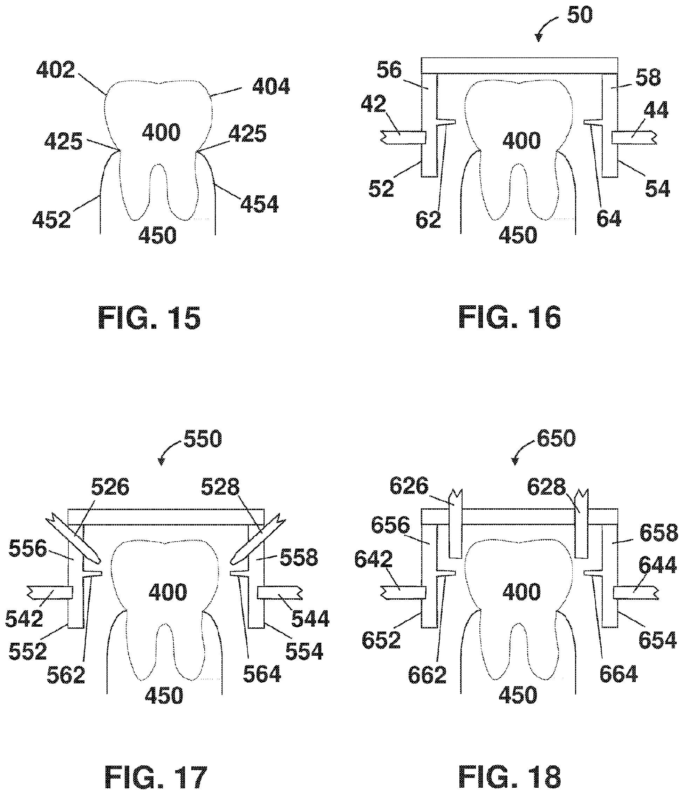

Entrained fluid pulses are used to clean the teeth and gingival area from either first side or second side of appliance (**50, 150, 250** or **350**). Appliances for use in the present invention are now discussed. FIG. 15 is a cross-sectional view of a mammalian tooth in the gums of the oral cavity. The figure shows tooth **400** with first side **402** and second side **404,** as well as gum **450** with first side **452** and second side **454.** Gum line **425** is at the point of intersection of tooth **400** and gum **450.**

FIG. 16 is a cross-sectional view of a section of a first embodiment of an appliance **50** used according to the present invention. Appliance **50** has first side **52** and second side **54,** first manifold **56** and second manifold **58,** and first jet **62** and second jet **64.** Tubes **42** and **44** convey entrained fluid pulses fluid to appliance **50.** Tube **42** conveys fluid to the first side **52** of appliance **50,** delivering the entrained fluid pulses into first manifold **56.** Tube **44** conveys fluid to the second side **54** of appliance **50,** delivering the entrained fluid pulses into second manifold **58.** The manifolds **56, 58** deliver entrained fluid pulses to jets **62, 64,** respectively. Jet **62** delivers entrained fluid pulses to with first side **402** of tooth **400,** and/or first side **452** of gum **450,** and/or gum line **425.** Jet **64** delivers entrained fluid pulses to second side **404** of tooth **400,** and or second side **454** of gum **450,** and/or gum line **425.** In some embodiments, delivery of entrained fluid pulses to gum line **425** is preferred. The distance from the nozzle to tooth surface is some embodiments is about 0mm (touching the tooth surface) to about 10mm, or about 1mm to about 5mm.

It should be noted that although FIG. 16 shows only one each of jets **62** and second jet **64,** in some embodiments manifolds **56, 58** may deliver entrained fluid pulses to multiple jets on first side **52** and/or second side **54** of appliance **50.**

In use, entrained fluid pulses would reciprocate from side to side of appliance **50.** So, the cleaning operation would alternate from first side **402** to second side **404** of tooth **400,** and/or first side **452** to second side **454** of gum **450.** In some embodiments, during reciprocation, while first jet **62** delivers entrained fluid pulse to first side of the tooth or gum, second jet **64** simultaneously vacuums the liquid phase of the entrained fluid pulse from the second side of the tooth or gum. Then, while second jet **64** delivers entrained fluid pulse to second side of the tooth or gum, first jet **62** simultaneously vacuums the liquid phase of the entrained fluid pulse from the first side of the tooth or gum.

The description above of appliance **50** could also be used to describe appliances **150, 250,** or **350.** In all these embodiments, entrained fluid is created before the fluid enters the appliance. In some embodiments, entrained fluid is created after the fluid enters the appliance.

FIG. 17 is a cross-sectional view of a section of a second embodiment of an appliance **550** used according to the present invention. Appliance **550** has first side **552** and second side **554,** first manifold **556** and second manifold **558,** and first jet **562** and second jet **564.** Tubes **542** and **544** convey liquid to appliance **550.** Tube **542** conveys liquid to the first side **552** of appliance **550,** delivering the liquid, which may be pulsed, into first manifold **556.** Tube **544** conveys liquid to the second side **554** of appliance **550,** delivering the liquid, which may be pulsed, into second manifold **558.** The manifolds **556, 558** deliver liquid to jets **562, 564,** respectively. Jet **562** delivers liquid to with first side **402** of tooth **400,** and/or first side **452** of gum **450,** and/or gum line **425.** Jet **564** delivers liquid to second side **404** of tooth **400,** and or second side **454** of gum **450,** and/or gum line **425.** In some embodiments, delivery of entrained fluid pulses to gum line **425** is preferred.

Third jet **526** delivers pulses of gas, to first side **402** of tooth **400,** and/or first side **452** of gum **450,** and/or gum line **425.** Entrained fluid pulses are created at the intersection of the gas flow from jet **526** and the liquid flow from jet **562.** Likewise, fourth jet **528** delivers pulses of gas, to second side **404** of tooth **400,** and/or second side **454** of gum **450,** and/or gum line **425.** Entrained fluid pulses are created at the intersection of the gas flow from jet **528** and the liquid flow from jet **564.**

It should be noted that although FIG. 17 shows only one each of jets **562** and second jet **564,** in some embodiments manifolds **556, 558** may deliver liquid to multiple jets on first side **552** and/or second side **554** of appliance **550.**

It should also be noted that third jet **526** and fourth jet **528,** which both deliver gas, is preferably directed towards the surfaces being cleaned or treated.

In use, entrained fluid pulses would reciprocate from side to side of appliance **550.** So, the cleaning operation would alternate from first side **402** to second side **404** of tooth **400,** and/or first side **452** to second side **454** of gum **450.** In some embodiments, during reciprocation, while first jet **562** delivers liquid to first side of the tooth or gum, second jet **564** simultaneously vacuums the liquid phase of the entrained fluid pulse from the second side of the tooth or gum. Then, while second jet **564** delivers liquid to second side of the tooth or gum, first jet **562** simultaneously vacuums the liquid phase of the entrained fluid pulse from the first side of the tooth or gum.

FIG. 18 is a cross-sectional view of a section of a third embodiment of an appliance **650** used according to the present invention. Appliance **650** has first side **652** and second side **654,** first manifold **656** and second manifold **658,** and first jet **662** and second jet **664.** Tubes **642** and **644** convey gas to appliance **650.** Tube **642** conveys gas to the first side **652** of appliance **650,** delivering pulsed gas into first manifold **656.** Tube **644** conveys gas to the second side **654** of appliance **650,** delivering pulsed gas into second manifold **658.** The manifolds **656, 658** deliver gas to jets **662, 664,** respectively. Jet **662** delivers gas to with first side **402** of tooth **400,** and/or first side **452** of gum **450,** and/or gum line **425.** Jet **664** delivers gas to second side **404** of tooth **400,** and or second side **454** of gum **450,** and/or gum line **425.** In some embodiments, delivery of entrained fluid pulses to gum line **425** is preferred.

Tube **626** delivers liquid, or pulses of liquid, to first side **402** of tooth **400,** and/or first side **452** of gum **450,** and/or gum line **425.** Entrained fluid pulses are created at the intersection of the liquid flow from tube **626** and the gas flow from jet **662.** Likewise, tube **628** delivers liquid, or pulses of liquid, to second side **404** of tooth **400,** and/or second side **454** of gum **450,** and/or gum line **425.** Entrained fluid pulses are created at the intersection of the liquid flow from tube **628** and the gas flow from jet **664.**

It should be noted that although FIG. 18 shows only one each of first jet **662** and second jet **664,** in some embodiments manifolds **656, 658** may deliver entrained fluid pulses to multiple jets on first side **652** and/or second side **654** of appliance **650.**

It should be noted that although FIG. 18 shows tube **626** delivering liquid to the region between first jet **662** and tooth **400,** in some embodiments tube **626** may deliver liquid to manifold **656.** Likewise, tube **628** may deliver liquid to manifold **658.** In these embodiments, entrained fluid is created in manifolds **656** and **658.** Entrained fluid pulses are then delivered to tooth **400** through jets **662** and **664.**

In use, entrained fluid pulses would reciprocate from side to side of appliance **650.** So, the cleaning operation would alternate from first side **402** to second side **404** of tooth **400,** and/or first side **452** to second side **454** of gum **450.** In some embodiments, during reciprocation, while tube **626** delivers liquid to first side of the tooth or gum, tube **628** simultaneously vacuums the liquid phase of the entrained fluid pulse from the second side of the tooth or gum. Then, while tube **628** delivers liquid to the second side of the tooth or gum, tube **626** simultaneously vacuums the liquid phase of the entrained fluid pulse from the first side of the tooth or gum.

### Pulsing Parameters

As noted above, in certain embodiments, the system/device of the present invention is designed to pulse one fluid (gas/liquid) into the other (liquid/gas) to create entrained fluid that is directed to at least one nozzle. In such embodiments, applicants have recognized that any of a variety of pulsing parameters may be used. In certain embodiments, the frequency of gas pulses as measured graphically (the "gas pulse frequency") that are combined with liquid/fluid pulses, is from about 0.1 Hz to about 25 Hz, including from about 0.25 Hz to about 5 Hz, including about 1 Hz. In certain embodiments, the frequency of liquid or liquid-containing fluid pulses as measured graphically (the "fluid pulse frequency") that are combined with the gas pulses, is from about 0 Hz to about 50 Hz, including from about 5 Hz to about 30 Hz, including about 25 Hz. While any suitable fluid and gas pulse frequencies may be used in accord with the present invention, applicants have discovered that in certain preferred embodiments, improved benefits can be achieved using a ratio of fluid pulse frequency to gas pulse frequency of from greater than 0 to about 50, including from greater than 0 to about 25, from greater than 0 to about 15, or from greater than 0 to about 10.

Any of a variety of other system parameters may be used. In certain embodiments, the gas is provided from its source at a gas manifold pressure of about 1 to about 100 psi (about 6.89×10³ to about 6.89×10⁵ Pascal), including from about 5 to about 35 psi (about 3.45×10⁴ to about 2.41×10⁵ Pascal), including from about 5 to about 20 psi (about 3.45×10⁴ to about 1.38×10⁵ Pascal), including from about 10 to about 15 psi (about 6.89×10⁴ to about 1.03×10⁵ Pascal), including about 10 psi (about 6.89×10⁴ Pascal). In certain embodiments, the gas pulse width is from about 0.100 ms to about 500 ms, including from about 5 ms to about 100 ms, including about 30 ms. In certain embodiments, the system provides a fluid pulse of about 0.0001ml to about 1 ml per nozzle-pulse, including from about 0.001 to about 0.10 ml per nozzle-pulse.

### EXAMPLES

### Example 1:

The system **10** as tested, and represented in FIG. 1, consists of a fluid supply reservoir **20** (Model B501, Alloy Products Corp., Waukesha, WI), connected by a tube **22,** to a custom programmable reciprocating flow controller **40** capable of both pulsing and reciprocating fluid, liquid, or gas at frequencies 0-25 Hz with programmable pulse duty cycles of 0-100%. The controller also allowed programming of the total treatment time, the amount of time/number of pulses before reciprocating, and the number of reciprocations. The flow controller **40** conveyed the fluid to the appliance **50** though tubes **44** and/or **42** to the appliance sides **54** and/or **52,** respectively. The appliance **50** for testing purposes was designed to provide a twenty nozzle array of 500-micron diameter nozzles on each side of the appliance. Nozzles were spaced evenly in a rectilinear pattern with nozzles spacing in the vertical and horizontal directions equal to 1500-micron, center to center. During testing, the nozzle exit was located in a fixed position, such that the interior nozzle would impact the target relatively perpendicular to, and at the center of, the substrate to be treated or cleaned. The interior nozzles **62** and **64** are shown in FIG. 16. The fixed distance from the nozzle exits and the biofilm covered substrate was positioned at 3000 +/-500um. Pressure was supplied to the fluid supply reservoir from a pressurized nitrogen tank (head pressure), regulated at the tank to 150 psi (1.03×10⁶ Pascal). Additional pressure regulation occurred post tank but before the fluid supply reservoir **20,** using a pressure regulator (Model 44-2211-241, Tescom, Minneapolis, MN) and digital pressure indicator (60 psi (4.14×10⁵ Pascal), Weis, Solar Metrix, Holtsville, NY). This input pressure was used to energize the fluid within the fluid supply reservoir and propel it through the system. Instrumentation and data capture and analysis software was used to measure and record the actual parameters during each test utilizing pressure sensors. The software graphed the data output of pressure vs time to allow pressure, pulse frequency, pulse width/duty cycle, reciprocation frequency and total treatment duration to be extracted from each test. This information was saved in graphical form for analysis post session (pressure vs. time graphs). Data was collected at a resolution of at least 1 KHz (i.e. one "pressure vs. time" point) every millisecond and represented and captured by a pressure graph for each session /test. The instrumentation used consisted of pressure sensors (Model MLH050PGB06A, Honeywell, Morristown, NJ). Sensors were positioned in tubes **44** and **42** using ¼" ID barbed Tee fittings, and located within 3 inches (7.62 cm) of each of the appliance sides, **54** and **52.** The pressure transducers went to a National Instruments DAQ (Model #USB-6343, National Instruments, Austin, TX), which was then conveyed to a commercial laptop computer with a USB-3.0 connection running National Instruments Labview and customized program to both record and display the data. AEFP occurs when the fluid supply reservoir is filled with liquid to a level that allows AEFP, where gas and liquid are simultaneously mixed and evacuated from the reservoir and supplied to the flow controller, typically <200ml liquid. Note: All "tubes" as described above are 1/4" ID x 12" to 18" in length, and rated at a minimum of 150 psi (1.03 ×10⁶ Pascal) pressure rating. One way valves with cracking pressures of less than 2 psi (1.38×10⁴ Pascal) were optionally placed in tubes **22, 44,** and **42** to prevent backflow.

The flow controller also allows for a second optional input/output not shown, which was used for these experiments to provide a negative differential pressure via a vacuum pump (not shown) in the appliance side/nozzles opposite the AEFP delivery. This negative pressure was created via peristaltic pump (Model #74203-47, Cole Parmer, Vernon Hills, IL) for all experiments where negative pressure was used. The collected fluid was discarded and not reused.

A series of trials were run with pressurized liquid (di-H2O) in fluid supply reservoir **20** to create a controlled full fluid pulse to allow control of pressure, pulse frequency, pulse width, pulse velocity and fluid reciprocation. A vacuum was applied to the side of applicator nozzles not receiving the pulse to prevent fluid accumulation at the specimen. The setup conditions utilized for the experiment are listed below:

| | |
|---|---|
| - Head Pressure: | 36 psi (2.48 ×10⁵ Pascal) |
| - Appliance Manifold Pressure: | 15 psi (1.03 ×10⁵ Pascal) |
| - Frequency: | 25 Hz |
| - Pulse width: | 30 ms |
| - Applicator - Rigid 20 nozzle (x2) Distance: | 3 mm |
| - Reciprocation: | 15s x 2 |
| - Calculated average liquid/nozzle-pulse: | 55microliter/ nozzle-pulse |
| - Calculated average pulse velocity: | 10 +/- 2m/s |

(Note: The reciprocation unit is defined as the amount of time the fluid is pulsed in one direction by the number of directions. So, 15s x 2 signifies that the unit pulsed for 15 seconds in the first direction and then 15 seconds in the second direction for a total of 30 seconds of treatment.)

Biofilms were grown *in vivo* for 7-days on 3-mm human enamel chips affixed in a denture on the buccal side in human subjects. This was done to achieve representative human biofilm characteristics experienced in the human dentitia with significant biofilm adhesion and cohesion tenacity. After 7 days growth, the enamel chips with the biofilm were removed and placed in a tooth shaped testing fixture to be representative of the surrounding tooth geometry. In this example, seven enamel chips were used.

REVEAL 100-7940 red stain (Henry Schein, Inc., Melville, NY) was utilized to stain the biofilm following the instructed red dye staining procedure both before and after treatment. Post-treatment reduction in the biofilm thickness, also referred to as volumetric removal, can be determined by a reduction in the red dye signal intensity when compared to the pre-treated stained sample. This type of reduction as evidenced by the residual stain intensity on the specimen, and can range from light pink, indicating significant biofilm thickness reduction, to a bright red signaling little to no biofilm thickness reduction. The observed volumetric removal occurs due to cohesion failure within the biofilm, but maintains attachment to the specimen surface, or "cratering" in which the plaque is pushed away outward from a treated region representing by a crater like formation within the specimen. Adhesion failure, also referred to as binary removal, can be determined by the lack of stain on the post-stained specimen, resulting in predominantly white/whitish-grey visible areas on the specimen.

The cleaning rating was determined based on a scale of "poor to best". "Poor" represented no binary biofilm removal and only minor volumetric removal. "Best" represented completed binary removal from the specimen. In between the "poor to best" categories were "fair", "good", and "better" assessments. All were categorized through visual assessment of both volumetric and binary removal.

The visual cleaning ratings for the seven chips used in this test were considered to be either "poor" or "fair".

Another trial was run with pressurized liquid (di-H2O) in fluid supply reservoir 20 to create a controlled full fluid pulse to allow control of pressure, pulse frequency, pulse width, pulse velocity and fluid reciprocation. Vacuum was applied to the side of applicator nozzles not receiving the pulse. The setup conditions utilized for the experiment are listed below:

| | |
|---|---|
| - Head Pressure: | 55 psi (3.79×10⁵ Pascal) |
| - Appliance manifold pressure: | 23 psi (1.59×10⁵ Pascal) |
| - Frequency: | 25 Hz |
| - Pulse width: | 30 ms |
| - Applicator - Rigid 20 nozzle (x2) Distance: | 3 mm |
| - Reciprocation: | 5s x 4 |
| - Calculated average liquid/nozzle-pulse: | 120 microliter /nozzle-pulse |
| - Calculated average pulse velocity: | 21 m/s |

(Note: The reciprocation unit is defined as the amount of time the fluid is pulsed in one direction by the number of directions. So, 5s x 4 signifies that the unit pulsed for 5 seconds in the first direction and then 5 seconds in the second direction, then repeated the first and second pulsing for a total of 20 seconds of treatment.)

In-vitro biofilms, 48 hour S-mutans, were grown on a 5mm sintered enamel like HA disk, and then placed in a tooth shaped testing fixture to provide representative oral geometry surrounding the specimen.

Again, REVEAL 100-7940 red stain (Henry Schein, Inc., Melville, NY) was utilized to stain the biofilm following the instructed red dye staining procedure both before and after treatment, and the cleaning rating was determined based on the "poor to best" scale described above.

The visual cleaning rating for the chip used in this test was considered to be "fair". In this test, the fluid head pressure was increased by 50% from the process condition in the first (36 psi to 55 psi (2.48× 10⁵ Pascal to 3.79×10⁵ Pascal)), resulting in a calculated average pulse velocity (21 m/s vs 10 m/s), two times greater than that of the earlier test. Small amounts of adhesion failure were observed within the direct impact area of the jets.

Finally, a negative control trial was run with pressurized gas (Nitrogen) in fluid supply reservoir **20** to create a controlled gas pulse to allow control of pressure, pulse frequency, pulse width, and treatment duration. Vacuum was applied to the side of applicator nozzles not receiving the pulse. The setup conditions utilized for the experiment are listed below:

| | |
|---|---|
| - Gas Input Pressure: | 36 psi (2.48×10⁵ Pascal) |
| - Measured appliance manifold pressure: | 23 psi (1.59×10⁵ Pascal) |
| - Pulse Frequency: | 25 Hz |
| - Pulse width: | 30 ms |
| - Applicator - Rigid 20 nozzle (x2) Distance: | 3 mm |

(Note: the measured pressure in the appliance manifold was recorded to start at 28 psi (1.93×10⁵ Pascal), then gradually decrease due to the system dynamic of pressure recovery time to 14 psi (9.65×10⁴ Pascal) at the end of 10 seconds.)

In-vitro biofilms, 48 hour S-mutans, were grown on a 5mm sintered enamel like HA disk, and then placed in a tooth shaped testing fixture to provide representative oral geometry surrounding the specimen.

REVEAL 100-7940 red stain (Henry Schein, Inc., Melville, NY) was once again utilized to stain the biofilm following the instructed red dye staining procedure both before and after treatment, and the cleaning rating was determined based on the "poor to best" scale described above.

The visual cleaning rating for the chip used in this test was considered to be "poor". This indicated that the gas alone, even at high pressures, was insufficient to cause significant biofilm disruption and removal.

### Example 2:

The system **100** as tested, and represented in FIG. 2, consists of a liquid supply reservoir **120** (Model B501, Alloy Products Corp., Waukesha, WI), and a gas supply reservoir **130.** The liquid reservoir **120** supplies the liquid to tube **122,** while the gas reservoir **130** supplies the gas to tube **132** which combine at the intersection of tubes **122** and **132** to create an air entrained fluid. The fluid (combined liquid and gas) is then conducted through the tube **132** to the first input of the custom programmable reciprocating flow controller **140** capable of both pulsing and reciprocating the fluid at frequencies 0-25 Hz with programmable pulse duty cycles of 0-100%. The controller also allowed programming of the total treatment time, the amount of time/number of pulses before reciprocating, and the number of reciprocations. The flow controller also allows for a second optional input/output through tube **146,** which for these experiments, was used a negative differential pressure (versus the positive pressure supplied through input tube **122** via vacuum pump **180.** This negative pressure was created via peristaltic pump (Model #74203-47, Cole Parmer, Vernon Hills, IL) for all experiments where negative pressure was used. The flow controller **140** conveys the fluid to the appliance **150** though tubes **144** or **142,** and from the appliance **150** through tubes **144** or **142.** In essence a four way valve, where the fluid controller **140** input at tube **122** is fluidly connected to output at tube **142,** when output tube **182** is fluidly connected to tube **144.** Or, where the fluid controller **140** input at tube **122** is fluidly connected to tube **144,** when output tube **182** is fluidly connected to tube **142.** Mostly liquid would be pulled back through tube **182** through the pump /vacuum source **180,** through tube **184,** where it could be collected in a separate reservoir or discarded (not shown), or captured back into the liquid reservoir **120** as show.

The appliance **150** for testing purposes was designed to provide a twenty nozzle array of 500-micron diameter nozzles on each side of the appliance. Nozzles were spaced evenly in a rectilinear pattern with nozzles spacing in the vertical and horizontal directions equal to 1500-micron, center to center. During testing, the nozzle exit was located in a fixed position, such that the interior nozzle would impact the target relatively perpendicular to, and at the center of, the substrate to be treated or cleaned. The fixed distance from the nozzle exits and the biofilm covered substrate was positioned at 3000 +/-500um. Positive Pressure was supplied to the liquid supply reservoir **120** from a pressurized nitrogen tank (head pressure), regulated at the tank to 150 psi (1.03×10⁶ Pascal). Additional pressure regulation occurred post tank but before the fluid supply reservoir **120,** using a pressure regulator (Model 44-2211-241, Tescom, Minneapolis, MN) and digital pressure indicator (60 psi (4.14×10⁵ Pascal), Weis, Solar Metrix, Holtsville, NY). This input pressure was used to energize the liquid within the fluid supply reservoir and propel it through tube **122.** The gas reservoir **130** pressure was provided by an air tank, regulated to 150 psi (1.03×10⁶ Pascal). Additional pressure regulation occurred post air tank but before the gas reservoir **130,** using a pressure regulator (Model 44-2211-241, Tescom, Minneapolis, MN) and digital pressure indicator (60 psi (4.14×10⁵ Pascal), Weis, Solar Metrix, Holtsville, NY). This input pressure was used to energize the gas within the gas supply reservoir and propel it through tube **132** and then combine it in tube **132** with liquid from tube **122** to create the air entrained fluid. This was then input under positive pressure to the flow controller **140.** Liquid entrained flow rate was controlled by adjusting the head gas pressure using the specified regulator and optionally further controlled using mechanical flow rate control orifices (not shown) in tube **122.** Gas flow rate was controlled via the gas control regulator.

Instrumentation and data capture and analysis software was used to measure and record the actual parameters during each test utilizing pressure sensors. The software graphed the data output of pressure versus time to allow pressure, pulse frequency, pulse width/duty cycle, reciprocation frequency and total treatment duration to be extracted from each test. This information was saved in graphical form for analysis post session (pressure versus time graphs). Data was collected at a resolution of at least 1 KHz sampling rate (i.e. one "pressure versus time" point) every millisecond and represented and captured by a pressure graph for each session /test. The instrumentation used consisted of pressure sensors (Model MLH050PGB06A, Honeywell, Morristown, NJ). Sensors were positioned in tubes **144** and **142** using ¼" ID barbed Tee fittings, and located within 3 inches (7.62 cm) of each of the appliance sides, **154** and **152.** The pressure transducers data was collected through a National Instruments DAQ (Model #USB-6343, National Instruments, Austin, TX), which was then conveyed to a commercial laptop computer with a USB-3.0 connection running National Instruments Labview and customized program to both record and display the data. Note: All "tubes" as described above are 1/4" ID x 12 to 18" length, and rated at a minimum pressure rating of 150 psi (1.03×10⁶ Pascal).

One way valves with cracking pressures of approximately 1 psi (6.89×10³ Pascal) were optionally placed in tubes **122** and **132** to prevent backflow.

### Example 3:

The system **200** as tested, and represented in FIG. 3, consists of a liquid supply reservoir **220** (Model B501, Alloy Products Corp., Waukesha, WI), a gas supply reservoir **230** (compressed air tank), a custom reciprocating flow controller **240** and custom appliance **250.** The reciprocating fluid controller consisted of both custom-built and commercially available components that allow programming of both the pulse and direction (reciprocation) of flow for the both gas pulse and the liquid pulse, as well as allowing synchronous or asynchronous operation between the gas pulse and fluid pulse. This was accomplished by triggering the liquid pulse to occur at the start of the gas pulse (NI DAQ model #USB-6363, National Instruments, Austin, TX) connected to pressure sensors (Model MLH050PGB06A, Honeywell, Morristown, NJ). The liquid pulse width, frequency, and direction were controlled through Labview custom program that actuated the appropriate solenoid valve (Model 71215SN2MN00N0C111P3, Honeywell, Morristown, NJ) to control the pulsing direction and timing versus the gas pulse. The gas pulse width, frequency and direction were controlled via the custom rotary valve that is programmed and controlled through the Maxon EPOS software. For the experiments conducted, the liquid was pulsed or entrained into the gas pulse downstream of the gas pulse control, creating the air entrained fluid pulse (AEFP). The AEFP travels through tubes **244** and/or **242,** depending on the programmed direction of flow and into the appliance **250.** Note in another embodiment, the liquid and gas programmable controllers representing the reciprocating fluid controller **240,** can be reversed. In the reversed embodiment, the gas is supplied through the solenoid/Labview controlled section of the reciprocating fluid controller **240,** and the liquid was supplied through the custom rotary valve.

The reciprocating fluid controller is capable of pulsing both the liquid and the gas at frequencies of 0 to 25 Hz with programmable pulse duty cycles of 0-100% for both gas and liquid. The controller also allowed programming of the total treatment time, the amount of time/number of pulses before reciprocating, and the number of reciprocations. The flow controller also allows for a second optional input/output (not shown), which for these experiments, and connected to a vacuum pump (not shown) to create a negative differential pressure. This negative pressure was created via peristaltic pump (Model #74203-47, Cole Parmer, Vernon Hills, IL) for all experiments where negative pressure was used. The collected fluid was discarded and not reused.

The appliance **250** for testing purposes was designed to provide a twenty nozzle array of 500-micron diameter nozzles on each side of the appliance. Nozzles were spaced evenly in a rectilinear pattern with nozzles spacing in the vertical and horizontal directions equal to 1500-micron, center to center. During testing, the nozzle exit was located in a fixed position such that the interior nozzle would impact the target relatively perpendicular to, and at the center of, the substrate to be treated or cleaned. The fixed distance from the nozzle exits and the biofilm covered substrate was positioned at 3000 +/-500um. Positive Pressure was supplied to the liquid supply reservoir **220** from a pressurized nitrogen tank (head pressure), regulated at the tank to 150 psi (1.03×10⁶ Pascal). Additional pressure regulation occurred post tank but before the fluid supply reservoir **220,** using a pressure regulator (Model 44-2211-241, Tescom, Minneapolis, MN) and digital pressure indicator (60 psi (4.14×10⁵ Pascal), Weis, Solar Metrix, Holtsville, NY). This input pressure was used to energize the liquid within the fluid supply reservoir and propel it through tube **222.** The gas reservoir **230** pressure was provided by an air tank, regulated to 150 psi (1.03×10⁶ Pascal). Additional pressure regulation occurred post air tank but before the gas reservoir **230,** using a pressure regulator (Model 44-2211-241, Tescom, Minneapolis, MN) and digital pressure indicator (60 psi (4.14×10⁵ Pascal), Weis, Solar Metrix, Holtsville, NY). This input pressure was used to energize the gas within the gas supply reservoir and propel it through tube **232** to the fluid controller. Pressurized liquid is supplied from the liquid supply reservoir **220** through tube **222** to the fluid controller. The liquid and gas are controlled, directed by their respective control systems and then combined to create the AEFP. Liquid entrained flow rate was controlled by adjusting the head gas pressure using the specified regulator and optionally further controlled using mechanical flow rate control orifices just before entrainment into the gas to create air entrained fluid. Gas flow rate was controlled via the gas control regulator.

The energized pulse flows through tubes **242** and/or **244** to the appliance. Instrumentation and data capture and analysis software was used to measure and record the actual parameters during each test utilizing pressure sensors. The software graphed the data output of pressure versus time to allow pressure, pulse frequency, pulse width/duty cycle, reciprocation frequency and total treatment duration to be extracted from each test. This information was saved in graphical form for analysis post session (pressure versus time graphs). Data was collected at a resolution of at least 1 KHz sampling rate (i.e. one "pressure vs. time" point) every millisecond and represented and captured by a pressure graph for each session /test. The instrumentation used consisted of pressure sensors (Model MLH050PGB06A, Honeywell, Morristown, NJ). Sensors were positioned in tubes **244** and **242** using ¼" ID barbed Tee fittings, and located within 3 inches (7.62 cm) of each of the appliance sides, **254** and 252. The pressure transducers data was collected through a National Instruments DAQ (Model #USB-6363, National Instruments, Austin, TX), which was then conveyed to a commercial laptop computer with a USB-3.0 connection running National Instruments Labview and customized program to both record and display the data. Note: All "tubes" as described above are 1/4" ID x 12 to 18" length, and rated at a minimum pressure rating of 150 psi (1.03×10⁶ Pascal).

One way valves with cracking pressures of approximately 1 psi (6.89×10³ Pascal) were optionally placed in tubes **252** and **244,** to prevent backflow. The negative pressure was applied to the appliance side, **252** or **254,** of the appliance **250** that was not receiving the AEFP pulse.

A series of trials were run with liquid (di-H2O) in fluid supply reservoir **220** and nitrogen gas in supply reservoir **230** to create a controlled entrained fluid pulse (AEFP), allowing control of the fluid entrainment rate, gas pulse pressure, pulse frequency, pulse width, and fluid reciprocation. A vacuum was applied to the side of applicator nozzles not receiving the pulse to prevent fluid accumulation at the specimen. The setup conditions utilized for the experiment are listed below:

| | |
|---|---|
| - Head Pressure: | 10 psi (6.89×10⁴ Pascal) |
| - Appliance Manifold Pressure: | 8 psi (5.52×10⁴ Pascal) |
| - Frequency: | 25 Hz |
| - Pulse width: | 30 ms |
| - Applicator - Rigid 20 nozzle (x2) Distance: | 3 mm |
| - Reciprocation: | 15s x 2 |
| - Calculated average liquid/ nozzle-pulse: | 40microliter/nozzle-pulse |
| - Approximate liquid pulse velocity (HSVM): | 10 +/-2 m/s |

(Note: Velocity was measured post testing via pixel tracking using High Speed Video Microscope, or HSVM).

In operation, 160ml of liquid was entrained at a constant rate for first 8 seconds of operation in each direction. The remaining 7 seconds in each direction was primarily pulsed gas only.

Biofilms were grown *in vivo* for 7-days on 3-mm human enamel chips affixed in a denture on the buccal side in human subjects. This was done to achieve representative human biofilm characteristics experienced in the human dentitia with significant biofilm adhesion and cohesion tenacity. After 7 days growth, the enamel chips with the biofilm were removed and placed in a tooth shaped testing fixture to be representative of the surrounding tooth geometry. In this example, twelve enamel chips were used.

REVEAL 100-7940 red stain (Henry Schein, Inc., Melville, NY) was utilized to stain the biofilm following the instructed red dye staining procedure both before and after treatment. Post-treatment reduction in the biofilm thickness, also referred to as volumetric removal, can be determined by a reduction in the red dye signal intensity when compared to the pre-treated stained sample. This type of reduction as evidenced by the residual stain intensity on the specimen, and can range from light pink, indicating significant biofilm thickness reduction, to a bright red signaling little to no biofilm thickness reduction. The observed volumetric removal occurs due to cohesion failure within the biofilm, but maintains attachment to the specimen surface, or "cratering" in which the plaque is pushed away outward from a treated region representing by a crater like formation within the specimen. Adhesion failure, also referred to as binary removal, can be determined by the lack of stain on the post-stained specimen, resulting in predominantly white/whitish-grey visible areas on the specimen.

The cleaning rating was determined based on a scale of "poor to best". "Poor" represented no binary biofilm removal and only minor volumetric removal. "Best" represented completed binary removal from the specimen. In between the "poor to best" categories were "fair", "good", and "better" assessments. All were categorized through visual assessment of both volumetric and binary removal.

The visual cleaning rating for the twelve chips used in this test was considered to be "good/better". In this test, with the addition of the gas pulse input, and entraining liquid at a constant rate, the gas input pressure and liquid head pressure was decreased by more than two/thirds from the process conditions in Example 1 (55/36 psi (3.79×10⁵/2.48×10⁵ Pascal) down to 10 psi (6.89×10⁴ Pascal), with measured peak liquid particle velocities in the range of 10 +/- 2 m/s. The in-situ grown saliva biofilm disruption and removal results were significantly improved when compared to the two tests from Example 1.

Another series of trials were run with liquid formulation (di-H20 w/ 10% silica Tixosil 63:Dioxyde de silicium, SiO₂) in fluid supply reservoir **220** and nitrogen gas in supply reservoir **230** to create a controlled entrained fluid pulse (AEFP), allowing control of the fluid entrainment rate, gas pulse pressure, pulse frequency, pulse width, and fluid reciprocation. A vacuum was applied to the side of applicator nozzles not receiving the pulse to prevent fluid accumulation at the specimen. The SiO₂ was provided as an abrasive in the formulation. The setup conditions utilized for the experiment are listed below:

| | |
|---|---|
| - Head Pressure: | 10 psi (6.89×10⁴ Pascal) |
| - Appliance Manifold Pressure: | 7 psi (4.83×10⁴ Pascal) |
| - Frequency: | 25 Hz |
| - Pulse width: | 30 ms |
| - Applicator - Rigid 20 nozzle (x2) Distance: | 3 mm |
| - Reciprocation: | 15s x 2 |
| - Calculated average liquid/ nozzle-pulse: | 35microliter/ nozzle-pulse |
| - Approximate liquid pulse velocity (HSVM): | 10 +/-2 m/s |

(Note: Velocity was measured post testing via pixel tracking using High Speed Video Microscope, or HSVM).

In operation, 160ml of liquid was entrained at a constant rate for first 9 seconds of operation in each direction. The remaining 6 seconds in each direction was primarily pulsed gas only.

Biofilms were grown *in vivo* for 7-days on 3-mm human enamel chips affixed in a denture on the buccal side in human subjects as discussed earlier in this example. Also, REVEAL 100-7940 red stain (Henry Schein, Inc., Melville, NY) was utilized to stain the biofilm as discussed earlier, and the cleaning rating was determined based on a scale of "poor to best".

The visual cleaning rating for the ten chips used in this test was considered to be "better/best". In this test, an abrasive was added to the formulation. The abrasive improved the overall cleaning results when compared to all previous tests.

Still another series of trials were run with liquid (di-H2O) in fluid supply reservoir **220** and nitrogen gas in supply reservoir **230** to create a controlled entrained fluid pulse (AEFP), allowing control of the fluid entrainment rate and pulse frequency, gas pulse frequency, and pulse width. Fluid reciprocation was not utilized. A vacuum was applied to the side of applicator nozzles not receiving the pulse to prevent fluid accumulation at the specimen. The setup conditions utilized for the experiment are listed below:

| | |
|---|---|
| - Gas Head Pressure: | 20 psi (1.38×10⁵ Pascal) |
| - Liquid head Source Pressure: | 10 psi (6.89×10⁴ Pascal) |
| - Pulse width: | 30 ms |
| - Applicator - Rigid 20 nozzle (x2) Distance: | 3 mm |
| - Calculated average liquid/ nozzle-pulse: | 40ul+/-15ul per nozzle-pulse |

In this set of trials, the liquid pulse frequency and the gas pulse frequency were varied, and the liquid to gas pulse frequency ratios were calculated. The liquid pulse frequency ranged from 0 HZ (no liquid supplied) to 25 HZ, and the gas pulse frequency ranged from 0.5HZ to 25 HZ. In one trial, liquid was supplied at a constant rate, and the gas pulse frequency was 25 HZ. The time of treatment was also varied, and the total gas pulses were calculated and reported for each test.

In-vitro biofilms, 48 hour S-mutans, were grown on a 5mm sintered enamel like HA disk, and then placed in a tooth shaped testing fixture to provide representative oral geometry surrounding the specimen.

Again, REVEAL 100-7940 red stain (Henry Schein, Inc., Melville, NY) was utilized to stain the biofilm following the instructed red dye staining procedure both before and after treatment. Following the post treatment and digital imaging of the red stained sample, the specimens were then stained with a crystal violet stain (Harleco (EMD Chemicals), 65092A-95) to provide improved contrast for visual image analysis. The cleaning rating was determined based on the "poor to best" scale described in all previous examples.

Table 1shows the effect of liquid to gas pulse frequency ratio to observed cleaning.

**Table 1: Cleaning performance versus liquid to gas pulse frequency ratio.**

| Trial no. | Fluid pulse frequency (Hz) | Gas pulse frequency (Hz) | Fluid/Gas frequency ratio | Cleaning time (s) | Total number of gas pulses | Cleaning rating |
|---|---|---|---|---|---|---|
| 5* | Constant | 25 | --- | 15 | 375 | Best |
| 6 | 1 | 10 | 0.1 | 30 | 300 | Best |
| 7 | 25 | 20 | 1.3 | 15 | 300 | Best |
| 8 | 25 | 10 | 2.5 | 30 | 300 | Best |
| 9 | 5 | 1.5 | 3.3 | 120 | 180 | Best |
| 10 | 25 | 5 | 5 | 30 | 150 | Best |
| 11 | 5 | 0.5 | 10 | 30 | 15 | Better |
| 12 | 25 | 2 | 12.5 | 30 | 60 | Good |
| 13 | 10 | 0.5 | 20 | 30 | 15 | Fair |
| 14 | 25 | 0.5 | 50 | 30 | 15 | Fair |
| 15** | 0 | 25 | 0 | 10 | 250 | Poor |

| | | | | | | |
|---|---|---|---|---|---|---|
| * - No gas flow. * * - No liquid flow. | | | | | | |

Table 1 shows that there was a correlation between the Fluid/Gas pulse frequency ratio and the cleaning rating. When the fluid pulse/gas pulse ratio was below 10, the visual cleaning rating was "better" to "best" (translating to approximately 100% biofilm removal). Ratios above 10 provided "good" to "fair" results. So, a higher the gas pulse frequency (versus pulse frequency), the more improved the disruption and removal of the biofilm from the specimen.

### Example 4:

The system **300** as tested, and represented in FIG. 4, consists of a liquid supply reservoir **320** (Model B501, Alloy Products Corp., Waukesha, WI), a gas supply reservoir **330** (compressed air tank), a custom reciprocating flow controller **240** and custom appliance **350.** The reciprocating fluid controller consisted of both custom-designed/built and commercially available components that allow programming of both the pulse and direction (reciprocation) of flow for the both gas pulse and the liquid pulse, as well as allowing synchronous or asynchronous operation between the gas pulse and fluid pulse. This was accomplished by triggering the liquid pulse to occur at the start of the gas pulse (NI DAQ model #USB-6363, National Instruments, Austin, TX) connected to pressure sensors, Model MLH050PGB06A, Honeywell, Morristown, NJ). The liquid pulse width, frequency, and direction were controlled through Labview custom program that actuated the appropriate solenoid valve (Model 71215SN2MN00N0C111P3, Honeywell, Morristown, NJ) to control the pulsing direction and timing of the liquid pulse through tubes **342** and/or **344.** The gas pulse width, frequency and direction were controlled via the custom rotary valve that is programmed and controlled through the Maxon EPOS software. The gas pulse was delivered to the tubes **346** or **348,** depending on the reciprocation direction and the liquid pulse direction, such that if liquid pulse was being delivered to first side **352,** from tube **342,** then gas pulse was also delivered to the first side **352** from tube **346.** Conversely, if the liquid pulse was second side **354** from tube **344,** then the gas pulse would also be delivered to the second side **354,** through tube **348.** Also, gas and liquid could be supplied to both first side **352** and second side **354,** through tubes **342, 346, 344,** and **348** respectively. For the purpose of these experiments, gas and liquid were supplied to the first side **352** through tubes **342** and **346,** while fluid was removed from the second side **354** through tubes **344** by creating negative relative pressure using a peristaltic pump (Model #74203-47, Cole Parmer, Vernon Hills, IL).

The flow controller also allows for a second optional input/output (not shown), which was used for these experiments to provide a negative differential pressure via a vacuum pump (not shown) in the side opposite AEFP delivery. This negative pressure was created via the peristaltic pump for all experiments where negative pressure was used. The collected fluid was discarded and not reused. The reciprocating fluid controller is capable of pulsing at frequencies of 0 to 25 Hz with programmable pulse duty cycles of 0-100% for either gas and/or liquid. The controller also allowed programming of the total treatment time, the amount of time/number of pulses before reciprocating, and the number of reciprocations.

The appliance **350** that was used for testing purposes was designed to provide a twenty nozzle array of 500um dia. nozzles on each side of the appliance. Nozzles were spaced evenly in a rectilinear 4x5 pattern with nozzles spacing in the vertical and horizontal directions equal to 1500-micron, center to center. During testing, the nozzle exit was located in a fixed position, such that the interior nozzle would impact the target relatively perpendicular to, and at the center of the substrate to be treated or cleaned. Nozzles **562** and **564,** and **662** and **664,** in FIGS. 17 and 18, respectively, illustrate the direction and position relative to the teeth **400** of these nozzles. In addition, as separate set of nozzles **526, 528,** and **626** and **628,** as depicted in FIGS. 17 and 18, respectively, and aligned vertically with the nozzles columns represented by nozzles **542, 544, 642,** and **644.** The fixed distance from the nozzle exits and the biofilm covered substrate was positioned at approximately 3000 microns. During the experimental testing, the air pulse was supplied to the teeth through nozzle array represented in FIG. 18 by nozzle **662,** and the liquid was entrained into the gas pulse through nozzle array **662,** to create the AEFP prior to contacting the teeth. Vacuum was applied to the opposite side during the experiments for removal of fluid through nozzle array on the opposite side, represented by nozzle **644.** Conversely, fluid could be injected/entrained into the appliance chamber through nozzles **562** and/or **544** as shown in FIG. 17, with the gas pulse supplied via nozzle **526** and/or **528,** to create the AEFP prior to tooth impact.

Positive Pressure was supplied to the liquid supply reservoir **320** from a pressurized nitrogen tank (head pressure), regulated at the tank to 150 psi (1.03×10⁶ Pascal). Additional pressure regulation occurred post tank but before the fluid supply reservoir **320** using a pressure regulator (Model 44-2211-241, Tescom, Minneapolis, MN) and digital pressure indicator (60 psi (4.14×10⁵ Pascal), Weis, Solar Metrix, Holtsville, NY). This input pressure was used to energize the liquid within the fluid supply reservoir and propel it through tube **322.** Gas reservoir **330** pressure was provided by an air tank, regulated to 150 psi (1.03×10⁶ Pascal). Additional pressure regulation occurred post air tank but before the gas reservoir **330,** using a pressure regulator (Model 44-2211-241, Tescom, Minneapolis, MN) and digital pressure indicator (60 psi (4.14×10⁵ Pascal), Weis, Solar Metrix, Holtsville, NY). This input pressure was used to energize the gas within the gas supply reservoir and propel it through to the fluid controller through tube **332.** Pressurized liquid is supplied from the liquid supply reservoir **320** through tube **322** to the fluid controller.

Liquid entrained flow rate was controlled by adjusting the head gas pressure using the specified regulator and optionally further controlled using mechanical flow rate control orifices (not shown) in tubes **322,** or **344** and **342.** Gas flow rate was controlled via the gas control regulator.

The liquid and gas are controlled, directed by their respective control systems and then combined to create the AEFP, after exiting the nozzles in the appliance but before contacting the teeth. Instrumentation and data capture and analysis software was used to measure and record the actual parameters during each test utilizing pressure sensors. The software graphed the data output of pressure vs time to allow pressure, pulse frequency, pulse width/duty cycle, reciprocation frequency and total treatment duration to be extracted from each test. This information was saved in graphical form for analysis post session (pressure versus time graphs). Data was collected at a resolution of at least 1 KHz sampling rate (i.e. one "pressure vs. time" point) every millisecond and represented and captured by a pressure graph for each session /test. The instrumentation used consisted of pressure sensors (Model MLH050PGB06A, Honeywell, Morristown, NJ). Sensors were positioned within the tubes **342, 344, 346** and **348** using ¼" ID barbed Tee fittings, and located within 3 inches (7.62 cm) of each of the appliance sides, **354** and **352.** The pressure transducers data was collected through a National Instruments DAQ (Model #USB-6363, National Instruments, Austin, TX), which was then conveyed to a commercial laptop computer with a USB-3.0 connection running National Instruments Labview and customized program to both record and display the data. Note: All "tubes" as described above are 1/4" ID x 12 to 18" length, and rated at a minimum pressure rating of 150 psi (1.03×10⁶ Pascal).

One way valves with cracking pressures of approximately 1 psi (6.89×10³ Pascal) were optionally placed in tubes **252** and **244,** to prevent backflow. The negative pressure was applied to the appliance side, **252** or **254,** of appliance **250** that was not receiving the AEFP pulse.

## Claims

1. An oral care system (10, 100) comprising:
a. an appliance (50, 150) comprising a first and second plurality of nozzles (1132, 1134), said appliance configured to be held in the mouth of a user with said first and second plurality of nozzles in fluid communication with one or more surfaces (402, 404, 452, 454) of the user's oral cavity;
b. a source of gas,
c. a source of liquid, wherein said source of liquid and source of gas are separate sources (120, 130); and
d. a fluid controller (40, 140) for directing fluid to and removing fluid from said appliance; wherein said system is configured to produce a series of entrained fluid pulses from said sources of liquid and gas; direct such pulses through the first plurality of said nozzles in said appliance to said one or more surfaces of the user's oral cavity; and remove fluid from said appliance through the second plurality of nozzles to said fluid controller;
wherein such system is configured such that gas from said source of gas is pulsed from said fluid controller to said first plurality of nozzles and liquid from said source of liquid flows or is pulsed into the gas pulsed from the fluid controller to form gas-entrained fluid between said fluid controller and said nozzles.

2. The system of claim 1 wherein said liquid is pulsed into said gas pulsed from the fluid controller at a frequency ratio (liquid/gas) of from greater than zero to about 50.

3. The system of claim 2 wherein said frequency ratio (liquid/gas) of from greater than zero to about 15.

4. The system of claim 1 wherein said source of gas provides gas at a pressure of 6.89×10³ to 6.89×10⁵ Pascal (1 to 100 psi).

5. The system of claim 1 wherein said source of gas provides gas at a pressure of 3.45×10⁴ to 1.38×10⁵ Pascal (5 to 20 psi).

6. The system of claim 1 wherein said system provides a fluid pulse of about 0.001 to about 0.10 ml per nozzle-pulse.

7. The system of claim 1 wherein said fluid controller operates to alternately: (i) direct entrained fluid through said first plurality of nozzles while simultaneously applying negative pressure to said second plurality of nozzles to remove fluid from said appliance; and (ii) direct entrained fluid through said second plurality of nozzles while simultaneously applying negative pressure to said first plurality of nozzles to remove fluid from said appliance.

## Patentansprüche

1. Mundpflegesystem (10, 100), umfassend:
a. eine Vorrichtung (50, 150), die eine erste und eine zweite Vielzahl von Düsen (1132, 1134) umfasst, wobei die Vorrichtung dazu ausgelegt ist, im Mund eines Benutzers gehalten zu werden, wobei die erste und die zweite Vielzahl von Düsen in Fluidverbindung mit einer oder mehreren Oberflächen (402, 404, 452, 454) der Mundhöhle des Benutzers stehen;
b. eine Gasquelle,
c. eine Flüssigkeitsquelle, wobei die Flüssigkeitsquelle und die Gasquelle getrennte Quellen (120, 130) sind; und
d. eine Fluidsteuerung (40, 140) zum Leiten von Fluid zu und Abführen von Fluid aus der Vorrichtung; wobei das System dazu ausgelegt ist, eine Reihe von Impulsen an mitgerissenem Fluid aus der Flüssigkeits- und Gasquelle zu erzeugen; derartige Impulse durch die erste Vielzahl der Düsen in der Vorrichtung zu der einen oder den mehreren Oberflächen der Mundhöhle des Benutzers zu leiten; und Fluid aus der Vorrichtung durch die zweite Vielzahl von Düsen zu der Fluidsteuerung abzuführen; wobei ein derartiges System so ausgelegt ist, dass Gas aus der Gasquelle von der Fluidsteuerung der ersten Vielzahl von Düsen impulsartig zugeführt wird und Flüssigkeit aus der Flüssigkeitsquelle in das von der Fluidsteuerung impulsartig zugeführte Gas strömt oder diesem impulsartig zugeführt wird, um von Gas mitgerissenes Fluid zwischen der Fluidsteuerung und den Düsen zu bilden.

2. System nach Anspruch 1, wobei die Flüssigkeit dem Gas impulsartig zugeführt wird, das von der Fluidsteuerung mit einem Frequenzverhältnis (Flüssigkeit/Gas) von größer als Null bis etwa 50 impulsartig zugeführt wird.

3. System nach Anspruch 2, wobei das Frequenzverhältnis (Flüssigkeit/Gas) größer als Null bis etwa 15 beträgt.

4. System nach Anspruch 1, wobei die Gasquelle Gas mit einem Druck von 6,89×10³ bis 6,89×10⁵ Pascal (1 bis 100 psi) bereitstellt.

5. System nach Anspruch 1, wobei die Gasquelle Gas mit einem Druck von 3,45×10⁴ bis 1,38×10⁵ Pascal (5 bis 20 psi) bereitstellt.

6. System nach Anspruch 1, wobei das System einen Fluidimpuls von etwa 0,001 bis etwa 0,10 ml pro Düsenimpuls bereitstellt.

7. System nach Anspruch 1, wobei die Fluidsteuerung abwechselnd zu Folgendem betrieben wird: (i) Leiten von mitgerissenem Fluid durch die erste Vielzahl von Düsen bei gleichzeitigem Aufbringen von Unterdruck auf die zweite Vielzahl von Düsen, um Fluid aus der Vorrichtung abzuführen; und (ii) Leiten von mitgerissenem Fluid durch die zweite Vielzahl von Düsen bei gleichzeitigem Aufbringen von Unterdruck auf die erste Vielzahl von Düsen, um Fluid aus der Vorrichtung abzuführen.

## Revendications

1. Système de soin buccal (10, 100) comprenant :
a. un appareil (50, 150) comportant des première et seconde pluralités de buses (1132, 1134), ledit appareil étant conçu pour être tenu dans la bouche d'un utilisateur avec lesdites première et seconde pluralités de buses en communication fluidique avec une ou plusieurs surfaces (402, 404, 452, 454) de la cavité buccale de l'utilisateur ;
b. une source de gaz,
c. une source de liquide, ladite source de liquide et ladite source de gaz étant des sources séparées (120, 130) ; et
d. un régulateur de fluide (40, 140) pour acheminer du fluide vers ledit appareil et en retirer le fluide ; ledit système étant conçu pour produire une série d'impulsions de fluide entraîné provenant desdites sources de liquide et de gaz ; diriger ces impulsions à travers la première pluralité desdites buses dans ledit appareil vers lesdites une ou plusieurs surfaces de la cavité buccale de l'utilisateur ; et retirer le fluide dudit appareil à travers la seconde pluralité de buses vers ledit régulateur de fluide ;
un tel système étant conçu de sorte que du gaz provenant de ladite source de gaz soit pulsé depuis ledit régulateur de fluide vers ladite première pluralité de buses et que du liquide provenant de ladite source de liquide s'écoule ou soit pulsé dans le gaz pulsé depuis le régulateur de fluide pour former un fluide entraîné par gaz entre ledit régulateur de fluide et lesdites buses.

2. Système selon la revendication 1, ledit liquide étant pulsé dans ledit gaz pulsé depuis le régulateur de fluide à un rapport de fréquence (liquide/gaz) compris entre plus de zéro et environ 50.

3. Système selon la revendication 2, ledit rapport de fréquence (liquide/gaz) étant compris entre plus de zéro et environ 15.

4. Système selon la revendication 1, ladite source de gaz fournissant du gaz à une pression comprise entre 6,89 x 10³ et 6,89 x 10⁵ Pascal (1 à 100 psi).

5. Système selon la revendication 1, ladite source de gaz fournissant du gaz à une pression comprise entre 3,45 x 10⁴ et 1,38 x 10⁵ Pascal (5 à 20 psi).

6. Système selon la revendication 1, ledit système fournissant une impulsion de fluide comprise entre environ 0,001 et environ 0,10 ml par impulsion de buse.

7. Système selon la revendication 1, ledit régulateur de fluide fonctionnant de sorte à, alternativement : (i) acheminer le fluide entraîné à travers ladite première pluralité de buses tout en appliquant simultanément une pression négative à ladite seconde pluralité de buses pour retirer le fluide dudit appareil ; et (ii) acheminer le fluide entraîné à travers ladite seconde pluralité de buses tout en appliquant simultanément une pression négative à ladite première pluralité de buses pour retirer le fluide dudit appareil.
